# EUROPEAN PATENT APPLICATION

(11) **EP 3 173 961 A1**
(43) Date of publication of application: **31.05.2017**
(21) Application number: 15306886.1
(22) Date of filing: 27.11.2015
(51) Int. Cl.: G06F 19/22, G06F 19/24, G06N 3/12

(54) **METHOD FOR STORING USER DATA AND DECODING INFORMATION IN SYNTHESIZED OLIGOS, APPARATUS AND SUBSTANCE**

(71) Applicant: Thomson Licensing, 92130 Issy-les-Moulineaux (FR)
(72) Inventor: Huetter, Ingo, 30982 Pattensen (DE); Blawat, Meinolf, 30659 Hannover (DE); Chen, Xiaoming, 30659 Hannover (DE); Gaedke, Klaus, 30659 Hannover (DE)
(74) Representative: Huchet, Anne

(57) **Abstract**

Data stored in synthesized nucleic acid sequences can only be decoded if the information about the encoding strategy is still available at the time of decoding. A method and an apparatus for storing user data and decoding information data together in subsets of a plurality of synthesized oligos are provided. User data and decoding information required for decoding the user data are obtained (11). A first subset of code symbol sequences having encoded the user data and a second subset of code symbol sequences having encoded the decoding information are created (12) and a nucleic acid synthesizer is used to synthesize (13) corresponding first and second subsets of oligos, wherein the second oligo subset contains less oligos than the first, and the oligos of the second subset exhibit at least one specific property not occurring in any oligo of the first subset to identify the oligos containing decoding information.

SEQUENCE LISTING

<110> Thomson Licensing

<120> Method for Storing User Data and Decoding Information in Synthesized
Oligos, Apparatus and Substance

<130> PD140072-EPA

<140> EP15306886.1
<141> 2015-11-27

<160> 236

<170> BiSSAP 1.3

<210> 1
<211> 18
<212> DNA
<213> Artificial Sequence

<220>
<223> Example sequence with coding scheme exception at ninth position
      shown on page 8 in line31

<400> 1
atcgatcgta gctcgaga                                                    18


<210> 2
<211> 18
<212> DNA
<213> Artificial Sequence

<220>
<223> Example sequence with coding scheme exception at ninth position
      shown on page 8 in line32

<400> 2
cgatcgatgc gatctagc                                                    18


<210> 3
<211> 20
<212> DNA
<213> Artificial Sequence

<220>
<223> First example sequence with detectable coding scheme change shown
      on page 9 in line 23

<400> 3
tgaagtcatg tacctttaca                                                  20


<210> 4
<211> 20
<212> DNA
<213> Artificial Sequence

<220>
<223> Second example sequence with detectable coding scheme change
      shown on page 9 in line 23

<400> 4
ggacttactt gaacgtgaaa                                                   20


<210> 5
<211> 18
<212> DNA
<213> Artificial Sequence

<220>
<223> First example sequence with coding scheme exception at ninth
      position shown on page 13 in line 9

<400> 5
atcgatcgta gctcgaga                                                     18


<210> 6
<211> 18
<212> DNA
<213> Artificial Sequence

<220>
<223> Second example sequence with coding scheme exception at ninth
      position shown on page 13 in line 9

<400> 6
cgatcgatgc gatctagc                                                     18


<210> 7
<211> 20
<212> DNA
<213> Artificial Sequence

<220>
<223> Example sequence with detectable coding scheme change shown on
      page 14 in line 3

<400> 7
tgaagtcatg tacctttaca                                                   20


<210> 8
<211> 20
<212> DNA
<213> Artificial Sequence

<220>
<223> Example sequence with detectable coding scheme change shown on
      page 14 in line 4

<400> 8
ggacttactt gaacgtgaaa                                                   20


<210> 9
<211> 57
<212> DNA
<213> Artificial Sequence

<220>

<223> Sequence with address part and decoding information data part of
      a first example of sequences comprising information represented
      using a graphical representation, as shown in line 1 of Fig. 3a

<400> 9
atatatatgc gcgcgcgcgc gcgcgcgcgc gcgcgcgcgc gcgcgcgcgc gcgcgcg        57


<210> 10
<211> 57
<212> DNA
<213> Artificial Sequence

<220>
<223> Sequence with address part and decoding information data part of
      a first example of sequences comprising information represented
      using a graphical representation, as shown in line 2 of Fig. 3a

<400> 10
ctatatatgc gcgcgcgcgc gcgcgcgcgc gcgcgcgcgc gcgcgcgcgc gcgcgcg        57


<210> 11
<211> 57
<212> DNA
<213> Artificial Sequence

<220>
<223> Sequence with address part and decoding information data part of
      a first example of sequences comprising information represented
      using a graphical representation, as shown in line 3 of Fig. 3a

<400> 11
agatatatgc gcgcgcgcgc gcgcgcgcgc gcgcgcgcgc gcgcgcgcgc gcgcgcg        57


<210> 12
<211> 57
<212> DNA
<213> Artificial Sequence

<220>
<223> Sequence with address part and decoding information data part of
      a first example of sequences comprising information represented
      using a graphical representation, as shown in line 4 of Fig. 3a

<400> 12
cgatatatgc gcgcgcgcgc gcgcgcgcgc gcgcgcgcgc gcgcgcgcgc gcgcgcg        57


<210> 13
<211> 57
<212> DNA
<213> Artificial Sequence

<220>
<223> Sequence with address part and decoding information data part of
      a first example of sequences comprising information represented
      using a graphical representation, as shown in line 5 of Fig. 3a

<400> 13
atctatatgc gcgcgcgcgc gcgcgcgcgc gcgcgcgcgc gcgcgcgcgc gcgcgcg        57

<210> 14
<211> 57
<212> DNA
<213> Artificial Sequence

<220>
<223> Sequence with address part and decoding information data part of
      a first example of sequences comprising information represented
      using a graphical representation, as shown in line 6 of Fig. 3a

<400> 14
ctctatatgc gcgcgcgcgc gcgcgcgcgc gcgcgcgcgc gcgcgcgcgc gcgcgcg        57


<210> 15
<211> 57
<212> DNA
<213> Artificial Sequence

<220>
<223> Sequence with address part and decoding information data part of
      a first example of sequences comprising information represented
      using a graphical representation, as shown in line 7 of Fig. 3a

<400> 15
agctatatgc gcgcgatata tatatatata tatatatata tatatatata tcgcgcg        57


<210> 16
<211> 57
<212> DNA
<213> Artificial Sequence

<220>
<223> Sequence with address part and decoding information data part of
      a first example of sequences comprising information represented
      using a graphical representation, as shown in line 8 of Fig. 3a

<400> 16
cgctatatgc gcgcgatata tatatatata tatatatata tatatatata tcgcgcg        57


<210> 17
<211> 57
<212> DNA
<213> Artificial Sequence

<220>
<223> Sequence with address part and decoding information data part of
      a first example of sequences comprising information represented
      using a graphical representation, as shown in line 9 of Fig. 3a

<400> 17
atagatatgc gcgcgatata tatatatata tatatatata tatatatata tcgcgcg        57


<210> 18
<211> 57
<212> DNA
<213> Artificial Sequence

<220>

<223> Sequence with address part and decoding information data part of
      a first example of sequences comprising information represented
      using a graphical representation, as shown in line 10 of Fig. 3a

<400> 18
ctagatatgc gcgcgatata tatatatata tatatatata tatatatata tcgcgcg        57


<210> 19
<211> 57
<212> DNA
<213> Artificial Sequence

<220>
<223> Sequence with address part and decoding information data part of
      a first example of sequences comprising information represented
      using a graphical representation, as shown in line 11 of Fig. 3a

<400> 19
agagatatgc gcgcgatata tatatatata tatatatata tatatatata tcgcgcg        57


<210> 20
<211> 57
<212> DNA
<213> Artificial Sequence

<220>
<223> Sequence with address part and decoding information data part of
      a first example of sequences comprising information represented
      using a graphical representation, as shown in line 12 of Fig. 3a

<400> 20
cgagatatgc gcgcgatata gcgcgcgcgc gcgcgcgcgc gcgcgcgata tcgcgcg        57


<210> 21
<211> 57
<212> DNA
<213> Artificial Sequence

<220>
<223> Sequence with address part and decoding information data part of
      a first example of sequences comprising information represented
      using a graphical representation, as shown in line 13 of Fig. 3a

<400> 21
atcgatatgc gcgcgatatc gcgcgcgcgc gcgcgcgcgc gcgcgcgata tcgcgcg        57


<210> 22
<211> 57
<212> DNA
<213> Artificial Sequence

<220>
<223> Sequence with address part and decoding information data part of
      a first example of sequences comprising information represented
      using a graphical representation, as shown in line 14 of Fig. 3a

<400> 22
ctcgatatgc gcgcgatata gcgcgcgcgc gcgcgcgcgc gcgcgcgata tcgcgcg        57

<210> 23
<211> 57
<212> DNA
<213> Artificial Sequence

<220>
<223> Sequence with address part and decoding information data part of
      a first example of sequences comprising information represented
      using a graphical representation, as shown in line 15 of Fig. 3a

<400> 23
agcgatatgc gcgcgatatc gcgcgcgcgc gcgcgcgcgc gcgcgcgata tcgcgcg        57


<210> 24
<211> 57
<212> DNA
<213> Artificial Sequence

<220>
<223> Sequence with address part and decoding information data part of
      a first example of sequences comprising information represented
      using a graphical representation, as shown in line 16 of Fig. 3a

<400> 24
cgcgatatgc gcgcgatata gcgcgcgcgc gcgcgcgcgc gcgcgcgata tcgcgcg        57


<210> 25
<211> 57
<212> DNA
<213> Artificial Sequence

<220>
<223> Sequence with address part and decoding information data part of
      a first example of sequences comprising information represented
      using a graphical representation, as shown in line 17 of Fig. 3a

<400> 25
atatctatgc gcgcgatatc gcgcgcgcgc gcgcgcgcgc gcgcgcgata tcgcgcg        57


<210> 26
<211> 57
<212> DNA
<213> Artificial Sequence

<220>
<223> Sequence with address part and decoding information data part of
      a first example of sequences comprising information represented
      using a graphical representation, as shown in line 18 of Fig. 3a

<400> 26
ctatctatgc gcgcgatata gcgcgcgcgc gcgcgcgcgc gcgcgcgata tcgcgcg        57


<210> 27
<211> 57
<212> DNA
<213> Artificial Sequence

<220>

<223> Sequence with address part and decoding information data part of
      a first example of sequences comprising information represented
      using a graphical representation, as shown in line 19 of Fig. 3a

<400> 27
agatctatgc gcgcgatatc gcgcgcgcgc gcgcgcgcgc gcgcgcgata tcgcgcg        57


<210> 28
<211> 57
<212> DNA
<213> Artificial Sequence

<220>
<223> Sequence with address part and decoding information data part of
      a first example of sequences comprising information represented
      using a graphical representation, as shown in line 20 of Fig. 3a

<400> 28
cgatctatgc gcgcgatata gcgcgcgcgc gcgcgcgcgc gcgcgcgata tcgcgcg        57


<210> 29
<211> 57
<212> DNA
<213> Artificial Sequence

<220>
<223> Sequence with address part and decoding information data part of
      a first example of sequences comprising information represented
      using a graphical representation, as shown in line 21 of Fig. 3a

<400> 29
atctctatgc gcgcgatata gcgcgcgcgc gcgcgcgcgc gcgcgcgata tcgcgcg        57


<210> 30
<211> 57
<212> DNA
<213> Artificial Sequence

<220>
<223> Sequence with address part and decoding information data part of
      a first example of sequences comprising information represented
      using a graphical representation, as shown in line 22 of Fig. 3a

<400> 30
ctctctatgc gcgcgatata gcgcgcgcgc gcgcgcgcgc gcgcgcgata tcgcgcg        57


<210> 31
<211> 57
<212> DNA
<213> Artificial Sequence

<220>
<223> Sequence with address part and decoding information data part of
      a first example of sequences comprising information represented
      using a graphical representation, as shown in line 23 of Fig. 3a

<400> 31
agctctatgc gcgcgatata gcgcgcgcgc gcgcgcgcgc gcgcgcgata tcgcgcg        57

<210> 32
<211> 57
<212> DNA
<213> Artificial Sequence

<220>
<223> Sequence with address part and decoding information data part of
      a first example of sequences comprising information represented
      using a graphical representation, as shown in line 24 of Fig. 3a

<400> 32
cgctctatgc gcgcgctata gcgcgcgcgc gcgcgcgcgc gcgcgctata tcgcgcg        57


<210> 33
<211> 57
<212> DNA
<213> Artificial Sequence

<220>
<223> Sequence with address part and decoding information data part of
      a first example of sequences comprising information represented
      using a graphical representation, as shown in line 25 of Fig. 3a

<400> 33
atagctatgc gcgcgctata gcgcgcgcgc gcgcgcgcgc gcgcgctata tcgcgcg        57


<210> 34
<211> 57
<212> DNA
<213> Artificial Sequence

<220>
<223> Sequence with address part and decoding information data part of
      a first example of sequences comprising information represented
      using a graphical representation, as shown in line 26 of Fig. 3a

<400> 34
ctagctatgc gcgcgctata tcgcgcgcgc gcgcgcgcgc gcgcgctata gcgcgcg        57


<210> 35
<211> 57
<212> DNA
<213> Artificial Sequence

<220>
<223> Sequence with address part and decoding information data part of
      a first example of sequences comprising information represented
      using a graphical representation, as shown in line 27 of Fig. 3a

<400> 35
agagctatgc gcgcgctata tagcgcgcgc gcgcgcgcgc gcgcgatata gcgcgcg        57


<210> 36
<211> 57
<212> DNA
<213> Artificial Sequence

<220>

<223> Sequence with address part and decoding information data part of
      a first example of sequences comprising information represented
      using a graphical representation, as shown in line 28 of Fig. 3a

<400> 36
cgagctatgc gcgcgcgata tagcgcgcgc gcgcgcgcgc gcgctatata gcgcgcg        57


<210> 37
<211> 57
<212> DNA
<213> Artificial Sequence

<220>
<223> Sequence with address part and decoding information data part of
      a first example of sequences comprising information represented
      using a graphical representation, as shown in line 29 of Fig. 3a

<400> 37
atcgctatgc gcgcgcgata tatagcgcgc gcgcgcgcgc gcgatatatc gcgcgcg        57


<210> 38
<211> 57
<212> DNA
<213> Artificial Sequence

<220>
<223> Sequence with address part and decoding information data part of
      a first example of sequences comprising information represented
      using a graphical representation, as shown in line 30 of Fig. 3a

<400> 38
ctcgctatgc gcgcgcgcta tatatagcgc gcgcgcgcgc gatatatagc gcgcgcg        57


<210> 39
<211> 57
<212> DNA
<213> Artificial Sequence

<220>
<223> Sequence with address part and decoding information data part of
      a first example of sequences comprising information represented
      using a graphical representation, as shown in line 31 of Fig. 3a

<400> 39
agcgctatgc gcgcgcgcga tatatatata gcgcgcgata tatatatagc gcgcgcg        57


<210> 40
<211> 57
<212> DNA
<213> Artificial Sequence

<220>
<223> Sequence with address part and decoding information data part of
      a first example of sequences comprising information represented
      using a graphical representation, as shown in line 32 of Fig. 3a

<400> 40
cgcgctatgc gcgcgcgcgc tatatatata tatatatata tatatatcgc gcgcgcg        57

<210> 41
<211> 57
<212> DNA
<213> Artificial Sequence

<220>
<223> Sequence with address part and decoding information data part of
      a first example of sequences comprising information represented
      using a graphical representation, as shown in line 33 of Fig. 3a

<400> 41
atatagatgc gcgcgcgcgc gatatatata tatatatata tatatcgcgc gcgcgcg        57


<210> 42
<211> 57
<212> DNA
<213> Artificial Sequence

<220>
<223> Sequence with address part and decoding information data part of
      a first example of sequences comprising information represented
      using a graphical representation, as shown in line 34 of Fig. 3a

<400> 42
ctatagatgc gcgcgcgcgc gcgatatata tatatatata tatagcgcgc gcgcgcg        57


<210> 43
<211> 57
<212> DNA
<213> Artificial Sequence

<220>
<223> Sequence with address part and decoding information data part of
      a first example of sequences comprising information represented
      using a graphical representation, as shown in line 35 of Fig. 3a

<400> 43
agatagatgc gcgcgcgcgc gcgcgctata tatatatata tcgcgcgcgc gcgcgcg        57


<210> 44
<211> 57
<212> DNA
<213> Artificial Sequence

<220>
<223> Sequence with address part and decoding information data part of
      a first example of sequences comprising information represented
      using a graphical representation, as shown in line 36 of Fig. 3a

<400> 44
cgatagatgc gcgcgcgcgc gcgcgcgcgc tatatagcgc gcgcgcgcgc gcgcgcg        57


<210> 45
<211> 57
<212> DNA
<213> Artificial Sequence

<220>

<223> Sequence with address part and decoding information data part of
      a first example of sequences comprising information represented
      using a graphical representation, as shown in line 37 of Fig. 3a

<400> 45
atctagatgc gcgcgcgcgc gcgcgcgcgc gcgcgcgcgc gcgcgcgcgc gcgcgcg        57


<210> 46
<211> 57
<212> DNA
<213> Artificial Sequence

<220>
<223> Sequence with address part and decoding information data part of
      a first example of sequences comprising information represented
      using a graphical representation, as shown in line 38 of Fig. 3a

<400> 46
ctctagatgc gcgcgcgcgc gcgcgcgcgc gcgcgcgcgc gcgcgcgcgc gcgcgcg        57


<210> 47
<211> 57
<212> DNA
<213> Artificial Sequence

<220>
<223> Sequence with address part and decoding information data part of
      a first example of sequences comprising information represented
      using a graphical representation, as shown in line 39 of Fig. 3a

<400> 47
agctagatgc gcgcgcgcgc gcgcgcgcgc gcgcgcgcgc gcgcgcgcgc gcgcgcg        57


<210> 48
<211> 57
<212> DNA
<213> Artificial Sequence

<220>
<223> Sequence with address part and decoding information data part of
      a first example of sequences comprising information represented
      using a graphical representation, as shown in line 1 of Fig. 3b

<400> 48
cgctagatgc gcgcgcgcgc gcgcgcgcgc gcgcgcgcgc gcgcgcgcgc gcgcgcg        57


<210> 49
<211> 57
<212> DNA
<213> Artificial Sequence

<220>
<223> Sequence with address part and decoding information data part of
      a first example of sequences comprising information represented
      using a graphical representation, as shown in line 2 of Fig. 3b

<400> 49
atagagatgc gcgcgcgcgc gcgcgcgcgc gcgcgcgcgc gcgcgcgcgc gcgcgcg        57

<210> 50
<211> 57
<212> DNA
<213> Artificial Sequence

<220>
<223> Sequence with address part and decoding information data part of
      a first example of sequences comprising information represented
      using a graphical representation, as shown in line 3 of Fig. 3b

<400> 50
ctagagatgc gcgcgcgcgc gcgcgcgcgc gcgcgcgcgc gcgcgcgcgc gcgcgcg        57


<210> 51
<211> 57
<212> DNA
<213> Artificial Sequence

<220>
<223> Sequence with address part and decoding information data part of
      a first example of sequences comprising information represented
      using a graphical representation, as shown in line 4 of Fig. 3b

<400> 51
agagagatgc gcgcgatata tatatatata tatatatata tatatatata tcgcgcg        57


<210> 52
<211> 57
<212> DNA
<213> Artificial Sequence

<220>
<223> Sequence with address part and decoding information data part of
      a first example of sequences comprising information represented
      using a graphical representation, as shown in line 5 of Fig. 3b

<400> 52
cgagagatgc gcgcgatata tatatatata tatatatata tatatatata tcgcgcg        57


<210> 53
<211> 57
<212> DNA
<213> Artificial Sequence

<220>
<223> Sequence with address part and decoding information data part of
      a first example of sequences comprising information represented
      using a graphical representation, as shown in line 6 of Fig. 3b

<400> 53
atcgagatgc gcgcgatata tatatatata tatatatata tatatatata tcgcgcg        57


<210> 54
<211> 57
<212> DNA
<213> Artificial Sequence

<220>

<223> Sequence with address part and decoding information data part of
      a first example of sequences comprising information represented
      using a graphical representation, as shown in line 7 of Fig. 3b

<400> 54
ctcgagatgc gcgcgatata tatatatata tatatatata tatatatata tcgcgcg        57


<210> 55
<211> 57
<212> DNA
<213> Artificial Sequence

<220>
<223> Sequence with address part and decoding information data part of
      a first example of sequences comprising information represented
      using a graphical representation, as shown in line 8 of Fig. 3b

<400> 55
agcgagatgc gcgcgatata tatatatata tatatatata tatatatata tcgcgcg        57


<210> 56
<211> 57
<212> DNA
<213> Artificial Sequence

<220>
<223> Sequence with address part and decoding information data part of
      a first example of sequences comprising information represented
      using a graphical representation, as shown in line 9 of Fig. 3b

<400> 56
cgcgagatgc gcgcgcgcgc gcgcgcgcgc gcgcgcgcgc gctatatata gcgcgcg        57


<210> 57
<211> 57
<212> DNA
<213> Artificial Sequence

<220>
<223> Sequence with address part and decoding information data part of
      a first example of sequences comprising information represented
      using a graphical representation, as shown in line 10 of Fig. 3b

<400> 57
atatcgatgc gcgcgcgcgc gcgcgcgcgc gcgcgcgcgc gatatatatc gcgcgcg        57


<210> 58
<211> 57
<212> DNA
<213> Artificial Sequence

<220>
<223> Sequence with address part and decoding information data part of
      a first example of sequences comprising information represented
      using a graphical representation, as shown in line 11 of Fig. 3b

<400> 58
ctatcgatgc gcgcgcgcgc gcgcgcgcgc gcgcgcgcga tatatatcgc gcgcgcg        57

<210> 59
<211> 57
<212> DNA
<213> Artificial Sequence

<220>
<223> Sequence with address part and decoding information data part of
      a first example of sequences comprising information represented
      using a graphical representation, as shown in line 12 of Fig. 3b

<400> 59
agatcgatgc gcgcgcgcgc gcgcgcgcgc gcgcgcgcta tatatagcgc gcgcgcg        57


<210> 60
<211> 57
<212> DNA
<213> Artificial Sequence

<220>
<223> Sequence with address part and decoding information data part of
      a first example of sequences comprising information represented
      using a graphical representation, as shown in line 13 of Fig. 3b

<400> 60
cgatcgatgc gcgcgcgcgc gcgcgcgcgc gcgcgctata tatagcgcgc gcgcgcg        57


<210> 61
<211> 57
<212> DNA
<213> Artificial Sequence

<220>
<223> Sequence with address part and decoding information data part of
      a first example of sequences comprising information represented
      using a graphical representation, as shown in line 14 of Fig. 3b

<400> 61
atctcgatgc gcgcgcgcgc gcgcgcgcgc gcgcgatata tatcgcgcgc gcgcgcg        57


<210> 62
<211> 57
<212> DNA
<213> Artificial Sequence

<220>
<223> Sequence with address part and decoding information data part of
      a first example of sequences comprising information represented
      using a graphical representation, as shown in line 15 of Fig. 3b

<400> 62
ctctcgatgc gcgcgcgcgc gcgcgcgcgc gcgatatata tcgcgcgcgc gcgcgcg        57


<210> 63
<211> 57
<212> DNA
<213> Artificial Sequence

<220>

<223> Sequence with address part and decoding information data part of
      a first example of sequences comprising information represented
      using a graphical representation, as shown in line 16 of Fig. 3b

<400> 63
agctcgatgc gcgcgcgcgc gcgcgcgcgc gctatatata gcgcgcgcgc gcgcgcg        57


<210> 64
<211> 57
<212> DNA
<213> Artificial Sequence

<220>
<223> Sequence with address part and decoding information data part of
      a first example of sequences comprising information represented
      using a graphical representation, as shown in line 17 of Fig. 3b

<400> 64
cgctcgatgc gcgcgcgcgc gcgcgcgcgc tatatatagc gcgcgcgcgc gcgcgcg        57


<210> 65
<211> 57
<212> DNA
<213> Artificial Sequence

<220>
<223> Sequence with address part and decoding information data part of
      a first example of sequences comprising information represented
      using a graphical representation, as shown in line 18 of Fig. 3b

<400> 65
atagcgatgc gcgcgcgcgc gcgcgcgcga tatatatcgc gcgcgcgcgc gcgcgcg        57


<210> 66
<211> 57
<212> DNA
<213> Artificial Sequence

<220>
<223> Sequence with address part and decoding information data part of
      a first example of sequences comprising information represented
      using a graphical representation, as shown in line 19 of Fig. 3b

<400> 66
ctagcgatgc gcgcgcgcgc gcgcgcgata tatatcgcgc gcgcgcgcgc gcgcgcg        57


<210> 67
<211> 57
<212> DNA
<213> Artificial Sequence

<220>
<223> Sequence with address part and decoding information data part of
      a first example of sequences comprising information represented
      using a graphical representation, as shown in line 20 of Fig. 3b

<400> 67
agagcgatgc gcgcgcgcgc gcgcgctata tatagcgcgc gcgcgcgcgc gcgcgcg        57

<210> 68
<211> 57
<212> DNA
<213> Artificial Sequence

<220>
<223> Sequence with address part and decoding information data part of
      a first example of sequences comprising information represented
      using a graphical representation, as shown in line 21 of Fig. 3b

<400> 68
cgagcgatgc gcgcgcgcgc gcgctatata tagcgcgcgc gcgcgcgcgc gcgcgcg        57


<210> 69
<211> 57
<212> DNA
<213> Artificial Sequence

<220>
<223> Sequence with address part and decoding information data part of
      a first example of sequences comprising information represented
      using a graphical representation, as shown in line 22 of Fig. 3b

<400> 69
atcgcgatgc gcgcgcgcgc gcgatatata tcgcgcgcgc gcgcgcgcgc gcgcgcg        57


<210> 70
<211> 57
<212> DNA
<213> Artificial Sequence

<220>
<223> Sequence with address part and decoding information data part of
      a first example of sequences comprising information represented
      using a graphical representation, as shown in line 23 of Fig. 3b

<400> 70
ctcgcgatgc gcgcgcgcgc gatatatatc gcgcgcgcgc gcgcgcgcgc gcgcgcg        57


<210> 71
<211> 57
<212> DNA
<213> Artificial Sequence

<220>
<223> Sequence with address part and decoding information data part of
      a first example of sequences comprising information represented
      using a graphical representation, as shown in line 24 of Fig. 3b

<400> 71
agcgcgatgc gcgcgcgcgc tatatatagc gcgcgcgcgc gcgcgcgcgc gcgcgcg        57


<210> 72
<211> 57
<212> DNA
<213> Artificial Sequence

<220>

<223> Sequence with address part and decoding information data part of
      a first example of sequences comprising information represented
      using a graphical representation, as shown in line 25 of Fig. 3b

<400> 72
cgcgcgatgc gcgcgcgcta tatatagcgc gcgcgcgcgc gcgcgcgcgc gcgcgcg        57


<210> 73
<211> 57
<212> DNA
<213> Artificial Sequence

<220>
<223> Sequence with address part and decoding information data part of
      a first example of sequences comprising information represented
      using a graphical representation, as shown in line 26 of Fig. 3b

<400> 73
atatatctgc gcgcgcgata tatatcgcgc gcgcgcgcgc gcgcgcgcgc gcgcgcg        57


<210> 74
<211> 57
<212> DNA
<213> Artificial Sequence

<220>
<223> Sequence with address part and decoding information data part of
      a first example of sequences comprising information represented
      using a graphical representation, as shown in line 27 of Fig. 3b

<400> 74
ctatatctgc gcgcgatata tatcgcgcgc gcgcgcgcgc gcgcgcgcgc gcgcgcg        57


<210> 75
<211> 57
<212> DNA
<213> Artificial Sequence

<220>
<223> Sequence with address part and decoding information data part of
      a first example of sequences comprising information represented
      using a graphical representation, as shown in line 28 of Fig. 3b

<400> 75
agatatctgc gcgcgatata tatatatata tatatatata tatatatata tcgcgcg        57


<210> 76
<211> 57
<212> DNA
<213> Artificial Sequence

<220>
<223> Sequence with address part and decoding information data part of
      a first example of sequences comprising information represented
      using a graphical representation, as shown in line 29 of Fig. 3b

<400> 76
cgatatctgc gcgcgatata tatatatata tatatatata tatatatata tcgcgcg        57

<210> 77
<211> 57
<212> DNA
<213> Artificial Sequence

<220>
<223> Sequence with address part and decoding information data part of
      a first example of sequences comprising information represented
      using a graphical representation, as shown in line 30 of Fig. 3b

<400> 77
atctatctgc gcgcgatata tatatatata tatatatata tatatatata tcgcgcg        57


<210> 78
<211> 57
<212> DNA
<213> Artificial Sequence

<220>
<223> Sequence with address part and decoding information data part of
      a first example of sequences comprising information represented
      using a graphical representation, as shown in line 31 of Fig. 3b

<400> 78
ctctatctgc gcgcgatata tatatatata tatatatata tatatatata tcgcgcg        57


<210> 79
<211> 57
<212> DNA
<213> Artificial Sequence

<220>
<223> Sequence with address part and decoding information data part of
      a first example of sequences comprising information represented
      using a graphical representation, as shown in line 32 of Fig. 3b

<400> 79
agctatctgc gcgcgcgcgc gcgcgcgcgc gcgcgcgcgc gcgcgcgcgc gcgcgcg        57


<210> 80
<211> 57
<212> DNA
<213> Artificial Sequence

<220>
<223> Sequence with address part and decoding information data part of
      a first example of sequences comprising information represented
      using a graphical representation, as shown in line 33 of Fig. 3b

<400> 80
cgctatctgc gcgcgcgcgc gcgcgcgcgc gcgcgcgcgc gcgcgcgcgc gcgcgcg        57


<210> 81
<211> 57
<212> DNA
<213> Artificial Sequence

<220>

<223> Sequence with address part and decoding information data part of
      a first example of sequences comprising information represented
      using a graphical representation, as shown in line 34 of Fig. 3b

<400> 81
atagatctgc gcgcgcgcgc gcgcgcgcgc gcgcgcgcgc gcgcgcgcgc gcgcgcg        57


<210> 82
<211> 57
<212> DNA
<213> Artificial Sequence

<220>
<223> Sequence with address part and decoding information data part of
      a first example of sequences comprising information represented
      using a graphical representation, as shown in line 1 of Fig. 3c

<400> 82
ctagatctgc gcgcgcgcgc gcgcgcgcgc gcgcgcgcgc gcgcgcgcgc gcgcgcg        57


<210> 83
<211> 57
<212> DNA
<213> Artificial Sequence

<220>
<223> Sequence with address part and decoding information data part of
      a first example of sequences comprising information represented
      using a graphical representation, as shown in line 2 of Fig. 3c

<400> 83
agagatctgc gcgcgagcgc gcgcgcgcgc gcgcgcgcgc gcgcgcgcgc gcgcgcg        57


<210> 84
<211> 57
<212> DNA
<213> Artificial Sequence

<220>
<223> Sequence with address part and decoding information data part of
      a first example of sequences comprising information represented
      using a graphical representation, as shown in line 3 of Fig. 3c

<400> 84
cgagatctgc gcgcgatagc gcgcgcgcgc gcgcgcgcgc gcgcgcgcgc gcgcgcg        57


<210> 85
<211> 57
<212> DNA
<213> Artificial Sequence

<220>
<223> Sequence with address part and decoding information data part of
      a first example of sequences comprising information represented
      using a graphical representation, as shown in line 4 of Fig. 3c

<400> 85
atcgatctgc gcgcgatata tcgcgcgcgc gcgcgcgcgc gcgcgcgcgc gcgcgcg        57

<210> 86
<211> 57
<212> DNA
<213> Artificial Sequence

<220>
<223> Sequence with address part and decoding information data part of
      a first example of sequences comprising information represented
      using a graphical representation, as shown in line 5 of Fig. 3c

<400> 86
ctcgatctgc gcgcgatata tatagcgcgc gcgcgcgcgc gcgcgcgcgc gcgcgcg        57


<210> 87
<211> 57
<212> DNA
<213> Artificial Sequence

<220>
<223> Sequence with address part and decoding information data part of
      a first example of sequences comprising information represented
      using a graphical representation, as shown in line 6 of Fig. 3c

<400> 87
agcgatctgc gcgcgatata tatatagcgc gcgcgcgcgc gcgcgcgcgc gcgcgcg        57


<210> 88
<211> 57
<212> DNA
<213> Artificial Sequence

<220>
<223> Sequence with address part and decoding information data part of
      a first example of sequences comprising information represented
      using a graphical representation, as shown in line 7 of Fig. 3c

<400> 88
cgcgatctgc gcgcgctata tatatatatc gcgcgcgcgc gcgcgcgcgc gcgcgcg        57


<210> 89
<211> 57
<212> DNA
<213> Artificial Sequence

<220>
<223> Sequence with address part and decoding information data part of
      a first example of sequences comprising information represented
      using a graphical representation, as shown in line 8 of Fig. 3c

<400> 89
atatctctgc gcgcgcgcta tatatatata tagcgcgcgc gcgcgcgcgc gcgcgcg        57


<210> 90
<211> 57
<212> DNA
<213> Artificial Sequence

<220>

<223> Sequence with address part and decoding information data part of
      a first example of sequences comprising information represented
      using a graphical representation, as shown in line 9 of Fig. 3c

<400> 90
ctatctctgc gcgcgcgcgc gatatatata tatagcgcgc gcgcgcgcgc gcgcgcg        57


<210> 91
<211> 57
<212> DNA
<213> Artificial Sequence

<220>
<223> Sequence with address part and decoding information data part of
      a first example of sequences comprising information represented
      using a graphical representation, as shown in line 10 of Fig. 3c

<400> 91
agatctctgc gcgcgcgcgc gcgctatata tatatatcgc gcgcgcgcgc gcgcgcg        57


<210> 92
<211> 57
<212> DNA
<213> Artificial Sequence

<220>
<223> Sequence with address part and decoding information data part of
      a first example of sequences comprising information represented
      using a graphical representation, as shown in line 11 of Fig. 3c

<400> 92
cgatctctgc gcgcgcgcgc gcgcgctata tatatatatc gcgcgcgcgc gcgcgcg        57


<210> 93
<211> 57
<212> DNA
<213> Artificial Sequence

<220>
<223> Sequence with address part and decoding information data part of
      a first example of sequences comprising information represented
      using a graphical representation, as shown in line 12 of Fig. 3c

<400> 93
atctctctgc gcgcgcgcgc gcgcgctata tatatatata tagcgcgcgc gcgcgcg        57


<210> 94
<211> 57
<212> DNA
<213> Artificial Sequence

<220>
<223> Sequence with address part and decoding information data part of
      a first example of sequences comprising information represented
      using a graphical representation, as shown in line 13 of Fig. 3c

<400> 94
ctctctctgc gcgcgcgcgc gcgcgctata gctatatata tatatcgcgc gcgcgcg        57

<210> 95
<211> 57
<212> DNA
<213> Artificial Sequence

<220>
<223> Sequence with address part and decoding information data part of
      a first example of sequences comprising information represented
      using a graphical representation, as shown in line 14 of Fig. 3c

<400> 95
agctctctgc gcgcgcgcgc gcgcgctata gcgcgatata tatatatcgc gcgcgcg        57


<210> 96
<211> 57
<212> DNA
<213> Artificial Sequence

<220>
<223> Sequence with address part and decoding information data part of
      a first example of sequences comprising information represented
      using a graphical representation, as shown in line 15 of Fig. 3c

<400> 96
cgctctctgc gcgcgcgcgc gcgcgctata gcgcgcgcta tatatatata gcgcgcg        57


<210> 97
<211> 57
<212> DNA
<213> Artificial Sequence

<220>
<223> Sequence with address part and decoding information data part of
      a first example of sequences comprising information represented
      using a graphical representation, as shown in line 16 of Fig. 3c

<400> 97
atagctctgc gcgcgcgcgc gcgcgctata gcgcgcgcgc tatatatata tcgcgcg        57


<210> 98
<211> 57
<212> DNA
<213> Artificial Sequence

<220>
<223> Sequence with address part and decoding information data part of
      a first example of sequences comprising information represented
      using a graphical representation, as shown in line 17 of Fig. 3c

<400> 98
ctagctctgc gcgcgcgcgc gcgcgctata gcgcgcgcgc gcgatatata tcgcgcg        57


<210> 99
<211> 57
<212> DNA
<213> Artificial Sequence

<220>

<223> Sequence with address part and decoding information data part of
      a first example of sequences comprising information represented
      using a graphical representation, as shown in line 18 of Fig. 3c

<400> 99
agagctctgc gcgcgcgcgc gcgcgctata gcgcgcgcgc gcgcgcgata tcgcgcg        57


<210> 100
<211> 57
<212> DNA
<213> Artificial Sequence

<220>
<223> Sequence with address part and decoding information data part of
      a first example of sequences comprising information represented
      using a graphical representation, as shown in line 19 of Fig. 3c

<400> 100
cgagctctgc gcgcgcgcgc gcgcgctata gcgcgcgcgc gcgctatata tcgcgcg        57


<210> 101
<211> 57
<212> DNA
<213> Artificial Sequence

<220>
<223> Sequence with address part and decoding information data part of
      a first example of sequences comprising information represented
      using a graphical representation, as shown in line 20 of Fig. 3c

<400> 101
atcgctctgc gcgcgcgcgc gcgcgctata gcgcgcgcgc gatatatata tcgcgcg        57


<210> 102
<211> 57
<212> DNA
<213> Artificial Sequence

<220>
<223> Sequence with address part and decoding information data part of
      a first example of sequences comprising information represented
      using a graphical representation, as shown in line 21 of Fig. 3c

<400> 102
ctcgctctgc gcgcgcgcgc gcgcgctata gcgcgcgcga tatatatata gcgcgcg        57


<210> 103
<211> 57
<212> DNA
<213> Artificial Sequence

<220>
<223> Sequence with address part and decoding information data part of
      a first example of sequences comprising information represented
      using a graphical representation, as shown in line 22 of Fig. 3c

<400> 103
agcgctctgc gcgcgcgcgc gcgcgctata gcgcgctata tatatatagc gcgcgcg        57

<210> 104
<211> 57
<212> DNA
<213> Artificial Sequence

<220>
<223> Sequence with address part and decoding information data part of
      a first example of sequences comprising information represented
      using a graphical representation, as shown in line 23 of Fig. 3c

<400> 104
cgcgctctgc gcgcgcgcgc gcgcgctata gcgatatata tatatcgcgc gcgcgcg        57


<210> 105
<211> 57
<212> DNA
<213> Artificial Sequence

<220>
<223> Sequence with address part and decoding information data part of
      a first example of sequences comprising information represented
      using a graphical representation, as shown in line 24 of Fig. 3c

<400> 105
atatagctgc gcgcgcgcgc gcgcgctata tatatatata tatcgcgcgc gcgcgcg        57


<210> 106
<211> 57
<212> DNA
<213> Artificial Sequence

<220>
<223> Sequence with address part and decoding information data part of
      a first example of sequences comprising information represented
      using a graphical representation, as shown in line 25 of Fig. 3c

<400> 106
ctatagctgc gcgcgcgcgc gcgcgctata tatatatata tcgcgcgcgc gcgcgcg        57


<210> 107
<211> 57
<212> DNA
<213> Artificial Sequence

<220>
<223> Sequence with address part and decoding information data part of
      a first example of sequences comprising information represented
      using a graphical representation, as shown in line 26 of Fig. 3c

<400> 107
agatagctgc gcgcgcgcgc gcgcgatata tatatatagc gcgcgcgcgc gcgcgcg        57


<210> 108
<211> 57
<212> DNA
<213> Artificial Sequence

<220>

<223> Sequence with address part and decoding information data part of
      a first example of sequences comprising information represented
      using a graphical representation, as shown in line 27 of Fig. 3c

<400> 108
cgatagctgc gcgcgcgcgc gcgatatata tatatagcgc gcgcgcgcgc gcgcgcg        57


<210> 109
<211> 57
<212> DNA
<213> Artificial Sequence

<220>
<223> Sequence with address part and decoding information data part of
      a first example of sequences comprising information represented
      using a graphical representation, as shown in line 28 of Fig. 3c

<400> 109
atctagctgc gcgcgcgcgc tatatatata tatcgcgcgc gcgcgcgcgc gcgcgcg        57


<210> 110
<211> 57
<212> DNA
<213> Artificial Sequence

<220>
<223> Sequence with address part and decoding information data part of
      a first example of sequences comprising information represented
      using a graphical representation, as shown in line 29 of Fig. 3c

<400> 110
ctctagctgc gcgcgcgata tatatatata tcgcgcgcgc gcgcgcgcgc gcgcgcg        57


<210> 111
<211> 57
<212> DNA
<213> Artificial Sequence

<220>
<223> Sequence with address part and decoding information data part of
      a first example of sequences comprising information represented
      using a graphical representation, as shown in line 29 of Fig. 3c

<400> 111
agctagctgc gcgcgatata tatatatagc gcgcgcgcgc gcgcgcgcgc gcgcgcg        57


<210> 112
<211> 57
<212> DNA
<213> Artificial Sequence

<220>
<223> Sequence with address part and decoding information data part of
      a first example of sequences comprising information represented
      using a graphical representation, as shown in line 31 of Fig. 3c

<400> 112
cgctagctgc gcgcgatata tatatagcgc gcgcgcgcgc gcgcgcgcgc gcgcgcg        57

<210> 113
<211> 57
<212> DNA
<213> Artificial Sequence

<220>
<223> Sequence with address part and decoding information data part of
      a first example of sequences comprising information represented
      using a graphical representation, as shown in line 32 of Fig. 3c

<400> 113
atagagctgc gcgcgatata tatcgcgcgc gcgcgcgcgc gcgcgcgcgc gcgcgcg        57


<210> 114
<211> 57
<212> DNA
<213> Artificial Sequence

<220>
<223> Sequence with address part and decoding information data part of
      a first example of sequences comprising information represented
      using a graphical representation, as shown in line 33 of Fig. 3c

<400> 114
ctagagctgc gcgcgatata tcgcgcgcgc gcgcgcgcgc gcgcgcgcgc gcgcgcg        57


<210> 115
<211> 57
<212> DNA
<213> Artificial Sequence

<220>
<223> Sequence with address part and decoding information data part of
      a first example of sequences comprising information represented
      using a graphical representation, as shown in line 34 of Fig. 3c

<400> 115
agagagctgc gcgcgatagc gcgcgcgcgc gcgcgcgcgc gcgcgcgcgc gcgcgcg        57


<210> 116
<211> 57
<212> DNA
<213> Artificial Sequence

<220>
<223> Sequence with address part and decoding information data part of
      a first example of sequences comprising information represented
      using a graphical representation, as shown in line 35 of Fig. 3c

<400> 116
cgagagctgc gcgcgagcgc gcgcgcgcgc gcgcgcgcgc gcgcgcgcgc gcgcgcg        57


<210> 117
<211> 57
<212> DNA
<213> Artificial Sequence

<220>

<223> Sequence with address part and decoding information data part of
      a first example of sequences comprising information represented
      using a graphical representation, as shown in line 36 of Fig. 3c

<400> 117
atcgagctgc gcgcgcgcgc gcgcgcgcgc gcgcgcgcgc gcgcgcgcgc gcgcgcg        57


<210> 118
<211> 57
<212> DNA
<213> Artificial Sequence

<220>
<223> Sequence with address part and decoding information data part of
      a first example of sequences comprising information represented
      using a graphical representation, as shown in line 37 of Fig. 3c

<400> 118
ctcgagctgc gcgcgcgcgc gcgcgcgcgc gcgcgcgcgc gcgcgcgcgc gcgcgcg        57


<210> 119
<211> 57
<212> DNA
<213> Artificial Sequence

<220>
<223> Sequence with address part and decoding information data part of
      a first example of sequences comprising information represented
      using a graphical representation, as shown in line 38 of Fig. 3c

<400> 119
agcgagctgc gcgcgcgcgc gcgcgcgcgc gcgcgcgcgc gcgcgcgcgc gcgcgcg        57


<210> 120
<211> 57
<212> DNA
<213> Artificial Sequence

<220>
<223> Sequence with address part and decoding information data part of
      a first example of sequences comprising information represented
      using a graphical representation, as shown in line 39 of Fig. 3c

<400> 120
cgcgagctgc gcgcgcgcgc gcgcgcgcgc gcgcgcgcgc gcgcgcgcgc gcgcgcg        57


<210> 121
<211> 57
<212> DNA
<213> Artificial Sequence

<220>
<223> Sequence with address part and decoding information data part of
      a first example of sequences comprising information represented
      using a graphical representation, as shown in line 40 of Fig. 3c

<400> 121
atatcgctgc gcgcgcgcgc gcgcgcgcgc gcgcgcgcgc gcgcgcgcgc gcgcgcg        57

<210> 122
<211> 57
<212> DNA
<213> Artificial Sequence

<220>
<223> Sequence with address part and decoding information data part of
      a first example of sequences comprising information represented
      using a graphical representation, as shown in line 41 of Fig. 3c

<400> 122
ctatcgctgc gcgcgcgcgc gcgcgcgcgc gcgcgcgcgc gcgcgcgcgc gcgcgcg        57


<210> 123
<211> 56
<212> DNA
<213> Artificial Sequence

<220>
<223> Sequence with address part and decoding information data part of
      a second example of sequences comprising information represented
      using a graphical representation, as shown in line 1 of Fig. 4a

<400> 123
ttaattaagg gcccgggccc gggcccgggc ccgggcccgg gcccgggccc gggccc        56


<210> 124
<211> 56
<212> DNA
<213> Artificial Sequence

<220>
<223> Sequence with address part and decoding information data part of
      a second example of sequences comprising information represented
      using a graphical representation, as shown in line 2 of Fig. 4a

<400> 124
gtaattaagg gcccgggccc gggcccgggc ccgggcccgg gcccgggccc gggccc        56


<210> 125
<211> 56
<212> DNA
<213> Artificial Sequence

<220>
<223> Sequence with address part and decoding information data part of
      a second example of sequences comprising information represented
      using a graphical representation, as shown in line 3 of Fig. 4a

<400> 125
tgaattaagg gcccgggccc gggcccgggc ccgggcccgg gcccgggccc gggccc        56


<210> 126
<211> 56
<212> DNA
<213> Artificial Sequence

<220>

<223> Sequence with address part and decoding information data part of
      a second example of sequences comprising information represented
      using a graphical representation, as shown in line 4 of Fig. 4a

<400> 126
ggaattaagg gcccgggccc gggcccgggc ccgggcccgg gcccgggccc gggccc          56


<210> 127
<211> 56
<212> DNA
<213> Artificial Sequence

<220>
<223> Sequence with address part and decoding information data part of
      a second example of sequences comprising information represented
      using a graphical representation, as shown in line 5 of Fig. 4a

<400> 127
ttcattaagg gcccgggccc gggcccgggc ccgggcccgg gcccgggccc gggccc          56


<210> 128
<211> 56
<212> DNA
<213> Artificial Sequence

<220>
<223> Sequence with address part and decoding information data part of
      a second example of sequences comprising information represented
      using a graphical representation, as shown in line 6 of Fig. 4a

<400> 128
gtcattaagg gcccgggccc gggcccgggc ccgggcccgg gcccgggccc gggccc          56


<210> 129
<211> 56
<212> DNA
<213> Artificial Sequence

<220>
<223> Sequence with address part and decoding information data part of
      a second example of sequences comprising information represented
      using a graphical representation, as shown in line 7 of Fig. 4a

<400> 129
tgcattaagg gcccgttaaa tttaaattta aatttaaatt taaatttaaa tggccc          56


<210> 130
<211> 56
<212> DNA
<213> Artificial Sequence

<220>
<223> Sequence with address part and decoding information data part of
      a second example of sequences comprising information represented
      using a graphical representation, as shown in line 8 of Fig. 4a

<400> 130
ggcattaagg gcccgttaaa tttaaattta aatttaaatt taaatttaaa tggccc          56

<210> 131
<211> 56
<212> DNA
<213> Artificial Sequence

<220>
<223> Sequence with address part and decoding information data part of
      a second example of sequences comprising information represented
      using a graphical representation, as shown in line 9 of Fig. 4a

<400> 131
ttacttaagg gcccgttaaa tttaaattta aatttaaatt taaatttaaa tggccc        56


<210> 132
<211> 56
<212> DNA
<213> Artificial Sequence

<220>
<223> Sequence with address part and decoding information data part of
      a second example of sequences comprising information represented
      using a graphical representation, as shown in line 10 of Fig. 4a

<400> 132
gtacttaagg gcccgttaaa tttaaattta aatttaaatt taaatttaaa tggccc        56


<210> 133
<211> 56
<212> DNA
<213> Artificial Sequence

<220>
<223> Sequence with address part and decoding information data part of
      a second example of sequences comprising information represented
      using a graphical representation, as shown in line 11 of Fig. 4a

<400> 133
tgacttaagg gcccgttaaa tttaaattta aatttaaatt taaatttaaa tggccc        56


<210> 134
<211> 56
<212> DNA
<213> Artificial Sequence

<220>
<223> Sequence with address part and decoding information data part of
      a second example of sequences comprising information represented
      using a graphical representation, as shown in line 12 of Fig. 4a

<400> 134
ggacttaagg gcccgttaaa gggcccgggc ccgggcccgg gcccgggaaa tggccc        56


<210> 135
<211> 56
<212> DNA
<213> Artificial Sequence

<220>

<223> Sequence with address part and decoding information data part of
      a second example of sequences comprising information represented
      using a graphical representation, as shown in line 13 of Fig. 4a

<400> 135
ttccttaagg gcccgttaac gggcccgggc ccgggcccgg gcccgggaaa tggccc          56


<210> 136
<211> 56
<212> DNA
<213> Artificial Sequence

<220>
<223> Sequence with address part and decoding information data part of
      a second example of sequences comprising information represented
      using a graphical representation, as shown in line 14 of Fig. 4a

<400> 136
gtccttaagg gcccgttaaa gggcccgggc ccgggcccgg gcccgggaaa tggccc          56


<210> 137
<211> 56
<212> DNA
<213> Artificial Sequence

<220>
<223> Sequence with address part and decoding information data part of
      a second example of sequences comprising information represented
      using a graphical representation, as shown in line 15 of Fig. 4a

<400> 137
tgccttaagg gcccgttaac gggcccgggc ccgggcccgg gcccgggaaa tggccc          56


<210> 138
<211> 56
<212> DNA
<213> Artificial Sequence

<220>
<223> Sequence with address part and decoding information data part of
      a second example of sequences comprising information represented
      using a graphical representation, as shown in line 16 of Fig. 4a

<400> 138
ggccttaagg gcccgttaaa gggcccgggc ccgggcccgg gcccgggaaa tggccc          56


<210> 139
<211> 56
<212> DNA
<213> Artificial Sequence

<220>
<223> Sequence with address part and decoding information data part of
      a second example of sequences comprising information represented
      using a graphical representation, as shown in line 17 of Fig. 4a

<400> 139
ttaagtaagg gcccgttaac gggcccgggc ccgggcccgg gcccgggaaa tggccc          56

<210> 140
<211> 56
<212> DNA
<213> Artificial Sequence

<220>
<223> Sequence with address part and decoding information data part of
      a second example of sequences comprising information represented
      using a graphical representation, as shown in line 18 of Fig. 4a

<400> 140
gtaagtaagg gcccgttaaa gggcccgggc ccgggcccgg gcccgggaaa tggccc        56


<210> 141
<211> 56
<212> DNA
<213> Artificial Sequence

<220>
<223> Sequence with address part and decoding information data part of
      a second example of sequences comprising information represented
      using a graphical representation, as shown in line 19 of Fig. 4a

<400> 141
tgaagtaagg gcccgttaac gggcccgggc ccgggcccgg gcccgggaaa tggccc        56


<210> 142
<211> 56
<212> DNA
<213> Artificial Sequence

<220>
<223> Sequence with address part and decoding information data part of
      a second example of sequences comprising information represented
      using a graphical representation, as shown in line 20 of Fig. 4a

<400> 142
ggaagtaagg gcccgttaaa gggcccgggc ccgggcccgg gcccgggaaa tggccc        56


<210> 143
<211> 56
<212> DNA
<213> Artificial Sequence

<220>
<223> Sequence with address part and decoding information data part of
      a second example of sequences comprising information represented
      using a graphical representation, as shown in line 21 of Fig. 4a

<400> 143
ttcagtaagg gcccgttaaa gggcccgggc ccgggcccgg gcccgggaaa tggccc        56


<210> 144
<211> 56
<212> DNA
<213> Artificial Sequence

<220>

<223> Sequence with address part and decoding information data part of
      a second example of sequences comprising information represented
      using a graphical representation, as shown in line 22 of Fig. 4a

<400> 144
gtcagtaagg gcccgttaaa gggcccgggc ccgggcccgg gcccgggaaa tggccc          56


<210> 145
<211> 56
<212> DNA
<213> Artificial Sequence

<220>
<223> Sequence with address part and decoding information data part of
      a second example of sequences comprising information represented
      using a graphical representation, as shown in line 23 of Fig. 4a

<400> 145
tgcagtaagg gcccgttaaa gggcccgggc ccgggcccgg gcccgggaaa tggccc          56


<210> 146
<211> 56
<212> DNA
<213> Artificial Sequence

<220>
<223> Sequence with address part and decoding information data part of
      a second example of sequences comprising information represented
      using a graphical representation, as shown in line 24 of Fig. 4a

<400> 146
ggcagtaagg gcccggtaaa gggcccgggc ccgggcccgg gcccggtaaa tggccc          56


<210> 147
<211> 56
<212> DNA
<213> Artificial Sequence

<220>
<223> Sequence with address part and decoding information data part of
      a second example of sequences comprising information represented
      using a graphical representation, as shown in line 25 of Fig. 4a

<400> 147
ttacgtaagg gcccggtaaa gggcccgggc ccgggcccgg gcccggtaaa tggccc          56


<210> 148
<211> 56
<212> DNA
<213> Artificial Sequence

<220>
<223> Sequence with address part and decoding information data part of
      a second example of sequences comprising information represented
      using a graphical representation, as shown in line 26 of Fig. 4a

<400> 148
gtacgtaagg gcccggtaaa tggcccgggc ccgggcccgg gcccggtaaa gggccc          56

<210> 149
<211> 56
<212> DNA
<213> Artificial Sequence

<220>
<223> Sequence with address part and decoding information data part of
      a second example of sequences comprising information represented
      using a graphical representation, as shown in line 27 of Fig. 4a

<400> 149
tgacgtaagg gcccggtaaa ttgcccgggc ccgggcccgg gcccgttaaa gggccc        56


<210> 150
<211> 56
<212> DNA
<213> Artificial Sequence

<220>
<223> Sequence with address part and decoding information data part of
      a second example of sequences comprising information represented
      using a graphical representation, as shown in line 28 of Fig. 4a

<400> 150
ggacgtaagg gcccgggaaa ttgcccgggc ccgggcccgg gccctttaaa gggccc        56


<210> 151
<211> 56
<212> DNA
<213> Artificial Sequence

<220>
<223> Sequence with address part and decoding information data part of
      a second example of sequences comprising information represented
      using a graphical representation, as shown in line 29 of Fig. 4a

<400> 151
ttccgtaagg gcccgggaaa tttaccgggc ccgggcccgg gccatttaac gggccc        56


<210> 152
<211> 56
<212> DNA
<213> Artificial Sequence

<220>
<223> Sequence with address part and decoding information data part of
      a second example of sequences comprising information represented
      using a graphical representation, as shown in line 30 of Fig. 4a

<400> 152
gtccgtaagg gcccgggcaa tttaaagggc ccgggcccgg gaaatttacc gggccc        56


<210> 153
<211> 56
<212> DNA
<213> Artificial Sequence

<220>

<223> Sequence with address part and decoding information data part of
      a second example of sequences comprising information represented
      using a graphical representation, as shown in line 31 of Fig. 4a

<400> 153
tgccgtaagg gcccgggcca tttaaattta ccgggccatt taaatttacc gggccc        56


<210> 154
<211> 56
<212> DNA
<213> Artificial Sequence

<220>
<223> Sequence with address part and decoding information data part of
      a second example of sequences comprising information represented
      using a graphical representation, as shown in line 32 of Fig. 4a

<400> 154
ggccgtaagg gcccgggccc tttaaattta aatttaaatt taaatttccc gggccc        56


<210> 155
<211> 56
<212> DNA
<213> Artificial Sequence

<220>
<223> Sequence with address part and decoding information data part of
      a second example of sequences comprising information represented
      using a graphical representation, as shown in line 33 of Fig. 4a

<400> 155
ttaatgaagg gcccgggccc gttaaattta aatttaaatt taaatggccc gggccc        56


<210> 156
<211> 56
<212> DNA
<213> Artificial Sequence

<220>
<223> Sequence with address part and decoding information data part of
      a second example of sequences comprising information represented
      using a graphical representation, as shown in line 34 of Fig. 4a

<400> 156
gtaatgaagg gcccgggccc gggaaattta aatttaaatt taaagggccc gggccc        56


<210> 157
<211> 56
<212> DNA
<213> Artificial Sequence

<220>
<223> Sequence with address part and decoding information data part of
      a second example of sequences comprising information represented
      using a graphical representation, as shown in line 35 of Fig. 4a

<400> 157
tgaatgaagg gcccgggccc gggcccttta aatttaaatt tcccgggccc gggccc        56

<210> 158
<211> 56
<212> DNA
<213> Artificial Sequence

<220>
<223> Sequence with address part and decoding information data part of
      a second example of sequences comprising information represented
      using a graphical representation, as shown in line 36 of Fig. 4a

<400> 158
ggaatgaagg gcccgggccc gggcccgggc aatttaccgg gcccgggccc gggccc        56


<210> 159
<211> 56
<212> DNA
<213> Artificial Sequence

<220>
<223> Sequence with address part and decoding information data part of
      a second example of sequences comprising information represented
      using a graphical representation, as shown in line 37 of Fig. 4a

<400> 159
ttcatgaagg gcccgggccc gggcccgggc ccgggcccgg gcccgggccc gggccc        56


<210> 160
<211> 56
<212> DNA
<213> Artificial Sequence

<220>
<223> Sequence with address part and decoding information data part of
      a second example of sequences comprising information represented
      using a graphical representation, as shown in line 38 of Fig. 4a

<400> 160
gtcatgaagg gcccgggccc gggcccgggc ccgggcccgg gcccgggccc gggccc        56


<210> 161
<211> 56
<212> DNA
<213> Artificial Sequence

<220>
<223> Sequence with address part and decoding information data part of
      a second example of sequences comprising information represented
      using a graphical representation, as shown in line 39 of Fig. 4a

<400> 161
tgcatgaagg gcccgggccc gggcccgggc ccgggcccgg gcccgggccc gggccc        56


<210> 162
<211> 56
<212> DNA
<213> Artificial Sequence

<220>

<223> Sequence with address part and decoding information data part of
      a second example of sequences comprising information represented
      using a graphical representation, as shown in line 1 of Fig. 4b

<400> 162
ggcatgaagg gcccgggccc gggcccgggc ccgggcccgg gcccgggccc gggccc        56


<210> 163
<211> 56
<212> DNA
<213> Artificial Sequence

<220>
<223> Sequence with address part and decoding information data part of
      a second example of sequences comprising information represented
      using a graphical representation, as shown in line 2 of Fig. 4b

<400> 163
ttactgaagg gcccgggccc gggcccgggc ccgggcccgg gcccgggccc gggccc        56


<210> 164
<211> 56
<212> DNA
<213> Artificial Sequence

<220>
<223> Sequence with address part and decoding information data part of
      a second example of sequences comprising information represented
      using a graphical representation, as shown in line 3 of Fig. 4b

<400> 164
gtactgaagg gcccgggccc gggcccgggc ccgggcccgg gcccgggccc gggccc        56


<210> 165
<211> 56
<212> DNA
<213> Artificial Sequence

<220>
<223> Sequence with address part and decoding information data part of
      a second example of sequences comprising information represented
      using a graphical representation, as shown in line 4 of Fig. 4b

<400> 165
tgactgaagg gcccgttaaa tttaaattta aatttaaatt taaatttaaa tggccc        56


<210> 166
<211> 56
<212> DNA
<213> Artificial Sequence

<220>
<223> Sequence with address part and decoding information data part of
      a second example of sequences comprising information represented
      using a graphical representation, as shown in line 5 of Fig. 4b

<400> 166
ggactgaagg gcccgttaaa tttaaattta aatttaaatt taaatttaaa tggccc        56

<210> 167
<211> 56
<212> DNA
<213> Artificial Sequence

<220>
<223> Sequence with address part and decoding information data part of
      a second example of sequences comprising information represented
      using a graphical representation, as shown in line 6 of Fig. 4b

<400> 167
ttcctgaagg gcccgttaaa tttaaattta aatttaaatt taaatttaaa tggccc        56


<210> 168
<211> 56
<212> DNA
<213> Artificial Sequence

<220>
<223> Sequence with address part and decoding information data part of
      a second example of sequences comprising information represented
      using a graphical representation, as shown in line 7 of Fig. 4b

<400> 168
gtcctgaagg gcccgttaaa tttaaattta aatttaaatt taaatttaaa tggccc        56


<210> 169
<211> 56
<212> DNA
<213> Artificial Sequence

<220>
<223> Sequence with address part and decoding information data part of
      a second example of sequences comprising information represented
      using a graphical representation, as shown in line 8 of Fig. 4b

<400> 169
tgcctgaagg gcccgttaaa tttaaattta aatttaaatt taaatttaaa tggccc        56


<210> 170
<211> 56
<212> DNA
<213> Artificial Sequence

<220>
<223> Sequence with address part and decoding information data part of
      a second example of sequences comprising information represented
      using a graphical representation, as shown in line 9 of Fig. 4b

<400> 170
ggcctgaagg gcccgggccc gggcccgggc ccgggcccgg gcaatttaaa gggccc        56


<210> 171
<211> 56
<212> DNA
<213> Artificial Sequence

<220>

<223> Sequence with address part and decoding information data part of
      a second example of sequences comprising information represented
      using a graphical representation, as shown in line 10 of Fig. 4b

<400> 171
ttaaggaagg gcccgggccc gggcccgggc ccgggcccgg gaaatttaac gggccc          56


<210> 172
<211> 56
<212> DNA
<213> Artificial Sequence

<220>
<223> Sequence with address part and decoding information data part of
      a second example of sequences comprising information represented
      using a graphical representation, as shown in line 11 of Fig. 4b

<400> 172
gtaaggaagg gcccgggccc gggcccgggc ccgggcccgt taaatttccc gggccc          56


<210> 173
<211> 56
<212> DNA
<213> Artificial Sequence

<220>
<223> Sequence with address part and decoding information data part of
      a second example of sequences comprising information represented
      using a graphical representation, as shown in line 12 of Fig. 4b

<400> 173
tgaaggaagg gcccgggccc gggcccgggc ccgggccctt taaattgccc gggccc          56


<210> 174
<211> 56
<212> DNA
<213> Artificial Sequence

<220>
<223> Sequence with address part and decoding information data part of
      a second example of sequences comprising information represented
      using a graphical representation, as shown in line 13 of Fig. 4b

<400> 174
ggaaggaagg gcccgggccc gggcccgggc ccgggcaatt taaagggccc gggccc          56


<210> 175
<211> 56
<212> DNA
<213> Artificial Sequence

<220>
<223> Sequence with address part and decoding information data part of
      a second example of sequences comprising information represented
      using a graphical representation, as shown in line 14 of Fig. 4b

<400> 175
ttcaggaagg gcccgggccc gggcccgggc ccgggaaatt taacgggccc gggccc          56

<210> 176
<211> 56
<212> DNA
<213> Artificial Sequence

<220>
<223> Sequence with address part and decoding information data part of
      a second example of sequences comprising information represented
      using a graphical representation, as shown in line 15 of Fig. 4b

<400> 176
gtcaggaagg gcccgggccc gggcccgggc ccgttaaatt tcccgggccc gggccc          56


<210> 177
<211> 56
<212> DNA
<213> Artificial Sequence

<220>
<223> Sequence with address part and decoding information data part of
      a second example of sequences comprising information represented
      using a graphical representation, as shown in line 16 of Fig. 4b

<400> 177
tgcaggaagg gcccgggccc gggcccgggc cctttaaatt gcccgggccc gggccc          56


<210> 178
<211> 56
<212> DNA
<213> Artificial Sequence

<220>
<223> Sequence with address part and decoding information data part of
      a second example of sequences comprising information represented
      using a graphical representation, as shown in line 17 of Fig. 4b

<400> 178
ggcaggaagg gcccgggccc gggcccgggc aatttaaagg gcccgggccc gggccc          56


<210> 179
<211> 56
<212> DNA
<213> Artificial Sequence

<220>
<223> Sequence with address part and decoding information data part of
      a second example of sequences comprising information represented
      using a graphical representation, as shown in line 18 of Fig. 4b

<400> 179
ttacggaagg gcccgggccc gggcccggga aatttaacgg gcccgggccc gggccc          56


<210> 180
<211> 56
<212> DNA
<213> Artificial Sequence

<220>

<223> Sequence with address part and decoding information data part of
      a second example of sequences comprising information represented
      using a graphical representation, as shown in line 19 of Fig. 4b

<400> 180
gtacggaagg gcccgggccc gggcccgtta aatttcccgg gcccgggccc gggccc        56


<210> 181
<211> 56
<212> DNA
<213> Artificial Sequence

<220>
<223> Sequence with address part and decoding information data part of
      a second example of sequences comprising information represented
      using a graphical representation, as shown in line 20 of Fig. 4b

<400> 181
tgacggaagg gcccgggccc gggcccttta aattgcccgg gcccgggccc gggccc        56


<210> 182
<211> 56
<212> DNA
<213> Artificial Sequence

<220>
<223> Sequence with address part and decoding information data part of
      a second example of sequences comprising information represented
      using a graphical representation, as shown in line 21 of Fig. 4b

<400> 182
ggacggaagg gcccgggccc gggcaattta aagggcccgg gcccgggccc gggccc        56


<210> 183
<211> 56
<212> DNA
<213> Artificial Sequence

<220>
<223> Sequence with address part and decoding information data part of
      a second example of sequences comprising information represented
      using a graphical representation, as shown in line 22 of Fig. 4b

<400> 183
ttccggaagg gcccgggccc gggaaattta acgggcccgg gcccgggccc gggccc        56


<210> 184
<211> 56
<212> DNA
<213> Artificial Sequence

<220>
<223> Sequence with address part and decoding information data part of
      a second example of sequences comprising information represented
      using a graphical representation, as shown in line 23 of Fig. 4b

<400> 184
gtccggaagg gcccgggccc gttaaatttc ccgggcccgg gcccgggccc gggccc        56

<210> 185
<211> 56
<212> DNA
<213> Artificial Sequence

<220>
<223> Sequence with address part and decoding information data part of
      a second example of sequences comprising information represented
      using a graphical representation, as shown in line 24 of Fig. 4b

<400> 185
tgccggaagg gcccgggccc tttaaattgc ccgggcccgg gcccgggccc gggccc        56


<210> 186
<211> 56
<212> DNA
<213> Artificial Sequence

<220>
<223> Sequence with address part and decoding information data part of
      a second example of sequences comprising information represented
      using a graphical representation, as shown in line 25 of Fig. 4b

<400> 186
ggccggaagg gcccgggcaa tttaaagggc ccgggcccgg gcccgggccc gggccc        56


<210> 187
<211> 56
<212> DNA
<213> Artificial Sequence

<220>
<223> Sequence with address part and decoding information data part of
      a second example of sequences comprising information represented
      using a graphical representation, as shown in line 26 of Fig. 4b

<400> 187
ttaattcagg gcccgggaaa tttaacgggc ccgggcccgg gcccgggccc gggccc        56


<210> 188
<211> 56
<212> DNA
<213> Artificial Sequence

<220>
<223> Sequence with address part and decoding information data part of
      a second example of sequences comprising information represented
      using a graphical representation, as shown in line 27 of Fig. 4b

<400> 188
gtaattcagg gcccgttaaa tttcccgggc ccgggcccgg gcccgggccc gggccc        56


<210> 189
<211> 56
<212> DNA
<213> Artificial Sequence

<220>

<223> Sequence with address part and decoding information data part of
      a second example of sequences comprising information represented
      using a graphical representation, as shown in line 28 of Fig. 4b

<400> 189
tgaattcagg gcccgttaaa tttaaattta aatttaaatt taaatttaaa tggccc        56


<210> 190
<211> 56
<212> DNA
<213> Artificial Sequence

<220>
<223> Sequence with address part and decoding information data part of
      a second example of sequences comprising information represented
      using a graphical representation, as shown in line 29 of Fig. 4b

<400> 190
ggaattcagg gcccgttaaa tttaaattta aatttaaatt taaatttaaa tggccc        56


<210> 191
<211> 56
<212> DNA
<213> Artificial Sequence

<220>
<223> Sequence with address part and decoding information data part of
      a second example of sequences comprising information represented
      using a graphical representation, as shown in line 30 of Fig. 4b

<400> 191
ttcattcagg gcccgttaaa tttaaattta aatttaaatt taaatttaaa tggccc        56


<210> 192
<211> 56
<212> DNA
<213> Artificial Sequence

<220>
<223> Sequence with address part and decoding information data part of
      a second example of sequences comprising information represented
      using a graphical representation, as shown in line 31 of Fig. 4b

<400> 192
gtcattcagg gcccgttaaa tttaaattta aatttaaatt taaatttaaa tggccc        56


<210> 193
<211> 56
<212> DNA
<213> Artificial Sequence

<220>
<223> Sequence with address part and decoding information data part of
      a second example of sequences comprising information represented
      using a graphical representation, as shown in line 32 of Fig. 4b

<400> 193
tgcattcagg gcccgggccc gggcccgggc ccgggcccgg gcccgggccc gggccc        56

<210> 194
<211> 56
<212> DNA
<213> Artificial Sequence

<220>
<223> Sequence with address part and decoding information data part of
      a second example of sequences comprising information represented
      using a graphical representation, as shown in line 33 of Fig. 4b

<400> 194
ggcattcagg gcccgggccc gggcccgggc ccgggcccgg gcccgggccc gggccc        56


<210> 195
<211> 56
<212> DNA
<213> Artificial Sequence

<220>
<223> Sequence with address part and decoding information data part of
      a second example of sequences comprising information represented
      using a graphical representation, as shown in line 34 of Fig. 4b

<400> 195
ttacttcagg gcccgggccc gggcccgggc ccgggcccgg gcccgggccc gggccc        56


<210> 196
<211> 56
<212> DNA
<213> Artificial Sequence

<220>
<223> Sequence with address part and decoding information data part of
      a second example of sequences comprising information represented
      using a graphical representation, as shown in line 1 of Fig. 4c

<400> 196
gtacttcagg gcccgggccc gggcccgggc ccgggcccgg gcccgggccc gggccc        56


<210> 197
<211> 56
<212> DNA
<213> Artificial Sequence

<220>
<223> Sequence with address part and decoding information data part of
      a second example of sequences comprising information represented
      using a graphical representation, as shown in line 2 of Fig. 4c

<400> 197
tgacttcagg gcccgtgccc gggcccgggc ccgggcccgg gcccgggccc gggccc        56


<210> 198
<211> 56
<212> DNA
<213> Artificial Sequence

<220>

<223> Sequence with address part and decoding information data part of
      a second example of sequences comprising information represented
      using a graphical representation, as shown in line 3 of Fig. 4c

<400> 198
ggacttcagg gcccgttacc gggcccgggc ccgggcccgg gcccgggccc gggccc          56


<210> 199
<211> 56
<212> DNA
<213> Artificial Sequence

<220>
<223> Sequence with address part and decoding information data part of
      a second example of sequences comprising information represented
      using a graphical representation, as shown in line 4 of Fig. 4c

<400> 199
ttccttcagg gcccgttaaa tggcccgggc ccgggcccgg gcccgggccc gggccc          56


<210> 200
<211> 56
<212> DNA
<213> Artificial Sequence

<220>
<223> Sequence with address part and decoding information data part of
      a second example of sequences comprising information represented
      using a graphical representation, as shown in line 5 of Fig. 4c

<400> 200
gtccttcagg gcccgttaaa tttaccgggc ccgggcccgg gcccgggccc gggccc          56


<210> 201
<211> 56
<212> DNA
<213> Artificial Sequence

<220>
<223> Sequence with address part and decoding information data part of
      a second example of sequences comprising information represented
      using a graphical representation, as shown in line 6 of Fig. 4c

<400> 201
tgccttcagg gcccgttaaa tttaaagggc ccgggcccgg gcccgggccc gggccc          56


<210> 202
<211> 56
<212> DNA
<213> Artificial Sequence

<220>
<223> Sequence with address part and decoding information data part of
      a second example of sequences comprising information represented
      using a graphical representation, as shown in line 7 of Fig. 4c

<400> 202
ggccttcagg gcccggtaaa tttaaatttc ccgggcccgg gcccgggccc gggccc          56

<210> 203
<211> 56
<212> DNA
<213> Artificial Sequence

<220>
<223> Sequence with address part and decoding information data part of
      a second example of sequences comprising information represented
      using a graphical representation, as shown in line 8 of Fig. 4c

<400> 203
ttaagtcagg gcccgggcaa tttaaattta aagggcccgg gcccgggccc gggccc        56


<210> 204
<211> 56
<212> DNA
<213> Artificial Sequence

<220>
<223> Sequence with address part and decoding information data part of
      a second example of sequences comprising information represented
      using a graphical representation, as shown in line 9 of Fig. 4c

<400> 204
gtaagtcagg gcccgggccc gttaaattta aattgcccgg gcccgggccc gggccc        56


<210> 205
<211> 56
<212> DNA
<213> Artificial Sequence

<220>
<223> Sequence with address part and decoding information data part of
      a second example of sequences comprising information represented
      using a graphical representation, as shown in line 10 of Fig. 4c

<400> 205
tgaagtcagg gcccgggccc gggcaattta aatttaacgg gcccgggccc gggccc        56


<210> 206
<211> 56
<212> DNA
<213> Artificial Sequence

<220>
<223> Sequence with address part and decoding information data part of
      a second example of sequences comprising information represented
      using a graphical representation, as shown in line 11 of Fig. 4c

<400> 206
ggaagtcagg gcccgggccc gggcccttta aatttaaatg gcccgggccc gggccc        56


<210> 207
<211> 56
<212> DNA
<213> Artificial Sequence

<220>

<223> Sequence with address part and decoding information data part of
      a second example of sequences comprising information represented
      using a graphical representation, as shown in line 12 of Fig. 4c

<400> 207
ttcagtcagg gcccgggccc gggcccttta aatttaaatt taccgggccc gggccc        56


<210> 208
<211> 56
<212> DNA
<213> Artificial Sequence

<220>
<223> Sequence with address part and decoding information data part of
      a second example of sequences comprising information represented
      using a graphical representation, as shown in line 13 of Fig. 4c

<400> 208
gtcagtcagg gcccgggccc gggcccttta cctttaaatt taaatggccc gggccc        56


<210> 209
<211> 56
<212> DNA
<213> Artificial Sequence

<220>
<223> Sequence with address part and decoding information data part of
      a second example of sequences comprising information represented
      using a graphical representation, as shown in line 14 of Fig. 4c

<400> 209
tgcagtcagg gcccgggccc gggcccttta ccgggaaatt taaatttccc gggccc        56


<210> 210
<211> 56
<212> DNA
<213> Artificial Sequence

<220>
<223> Sequence with address part and decoding information data part of
      a second example of sequences comprising information represented
      using a graphical representation, as shown in line 15 of Fig. 4c

<400> 210
ggcagtcagg gcccgggccc gggcccttta ccgggccctt taaatttaaa gggccc        56


<210> 211
<211> 56
<212> DNA
<213> Artificial Sequence

<220>
<223> Sequence with address part and decoding information data part of
      a second example of sequences comprising information represented
      using a graphical representation, as shown in line 16 of Fig. 4c

<400> 211
ttacgtcagg gcccgggccc gggcccttta ccgggcccgg taaatttaaa tggccc        56

<210> 212
<211> 56
<212> DNA
<213> Artificial Sequence

<220>
<223> Sequence with address part and decoding information data part of
      a second example of sequences comprising information represented
      using a graphical representation, as shown in line 17 of Fig. 4c

<400> 212
gtacgtcagg gcccgggccc gggcccttta ccgggcccgg gccatttaaa tggccc        56


<210> 213
<211> 56
<212> DNA
<213> Artificial Sequence

<220>
<223> Sequence with address part and decoding information data part of
      a second example of sequences comprising information represented
      using a graphical representation, as shown in line 18 of Fig. 4c

<400> 213
tgacgtcagg gcccgggccc gggcccttta ccgggcccgg gcccgggaaa tggccc        56


<210> 214
<211> 56
<212> DNA
<213> Artificial Sequence

<220>
<223> Sequence with address part and decoding information data part of
      a second example of sequences comprising information represented
      using a graphical representation, as shown in line 19 of Fig. 4c

<400> 214
ggacgtcagg gcccgggccc gggcccttta ccgggcccgg gccctttaaa tggccc        56


<210> 215
<211> 56
<212> DNA
<213> Artificial Sequence

<220>
<223> Sequence with address part and decoding information data part of
      a second example of sequences comprising information represented
      using a graphical representation, as shown in line 20 of Fig. 4c

<400> 215
ttccgtcagg gcccgggccc gggcccttta ccgggcccgg gaaatttaaa tggccc        56


<210> 216
<211> 56
<212> DNA
<213> Artificial Sequence

<220>

<223> Sequence with address part and decoding information data part of
      a second example of sequences comprising information represented
      using a graphical representation, as shown in line 21 of Fig. 4c

<400> 216
gtccgtcagg gcccgggccc gggcccttta ccgggcccgt taaatttaaa gggccc          56


<210> 217
<211> 56
<212> DNA
<213> Artificial Sequence

<220>
<223> Sequence with address part and decoding information data part of
      a second example of sequences comprising information represented
      using a graphical representation, as shown in line 22 of Fig. 4c

<400> 217
tgccgtcagg gcccgggccc gggcccttta ccgggcaatt taaatttacc gggccc          56


<210> 218
<211> 56
<212> DNA
<213> Artificial Sequence

<220>
<223> Sequence with address part and decoding information data part of
      a second example of sequences comprising information represented
      using a graphical representation, as shown in line 23 of Fig. 4c

<400> 218
ggccgtcagg gcccgggccc gggcccttta ccgttaaatt taaatggccc gggccc          56


<210> 219
<211> 56
<212> DNA
<213> Artificial Sequence

<220>
<223> Sequence with address part and decoding information data part of
      a second example of sequences comprising information represented
      using a graphical representation, as shown in line 24 of Fig. 4c

<400> 219
ttaatgcagg gcccgggccc gggcccttta aatttaaatt taacgggccc gggccc          56


<210> 220
<211> 56
<212> DNA
<213> Artificial Sequence

<220>
<223> Sequence with address part and decoding information data part of
      a second example of sequences comprising information represented
      using a graphical representation, as shown in line 25 of Fig. 4c

<400> 220
gtaatgcagg gcccgggccc gggcccttta aatttaaatt tcccgggccc gggccc          56

<210> 221
<211> 56
<212> DNA
<213> Artificial Sequence

<220>
<223> Sequence with address part and decoding information data part of
      a second example of sequences comprising information represented
      using a graphical representation, as shown in line 26 of Fig. 4c

<400> 221
tgaatgcagg gcccgggccc gggccattta aatttaaagg gcccgggccc gggccc        56


<210> 222
<211> 56
<212> DNA
<213> Artificial Sequence

<220>
<223> Sequence with address part and decoding information data part of
      a second example of sequences comprising information represented
      using a graphical representation, as shown in line 27 of Fig. 4c

<400> 222
ggaatgcagg gcccgggccc gggaaattta aatttaccgg gcccgggccc gggccc        56


<210> 223
<211> 56
<212> DNA
<213> Artificial Sequence

<220>
<223> Sequence with address part and decoding information data part of
      a second example of sequences comprising information represented
      using a graphical representation, as shown in line 28 of Fig. 4c

<400> 223
ttcatgcagg gcccgggccc tttaaattta aatggcccgg gcccgggccc gggccc        56


<210> 224
<211> 56
<212> DNA
<213> Artificial Sequence

<220>
<223> Sequence with address part and decoding information data part of
      a second example of sequences comprising information represented
      using a graphical representation, as shown in line 29 of Fig. 4c

<400> 224
gtcatgcagg gcccgggaaa tttaaattta acgggcccgg gcccgggccc gggccc        56


<210> 225
<211> 56
<212> DNA
<213> Artificial Sequence

<220>

<223> Sequence with address part and decoding information data part of
      a second example of sequences comprising information represented
      using a graphical representation, as shown in line 30 of Fig. 4c

<400> 225
tgcatgcagg gcccgttaaa tttaaattgc ccgggcccgg gcccgggccc gggccc          56


<210> 226
<211> 56
<212> DNA
<213> Artificial Sequence

<220>
<223> Sequence with address part and decoding information data part of
      a second example of sequences comprising information represented
      using a graphical representation, as shown in line 31 of Fig. 4c

<400> 226
ggcatgcagg gcccgttaaa tttaaagggc ccgggcccgg gcccgggccc gggccc          56


<210> 227
<211> 56
<212> DNA
<213> Artificial Sequence

<220>
<223> Sequence with address part and decoding information data part of
      a second example of sequences comprising information represented
      using a graphical representation, as shown in line 32 of Fig. 4c

<400> 227
ttactgcagg gcccgttaaa tttcccgggc ccgggcccgg gcccgggccc gggccc          56


<210> 228
<211> 56
<212> DNA
<213> Artificial Sequence

<220>
<223> Sequence with address part and decoding information data part of
      a second example of sequences comprising information represented
      using a graphical representation, as shown in line 33 of Fig. 4c

<400> 228
gtactgcagg gcccgttaaa tggcccgggc ccgggcccgg gcccgggccc gggccc          56


<210> 229
<211> 56
<212> DNA
<213> Artificial Sequence

<220>
<223> Sequence with address part and decoding information data part of
      a second example of sequences comprising information represented
      using a graphical representation, as shown in line 34 of Fig. 4c

<400> 229
tgactgcagg gcccgttacc gggcccgggc ccgggcccgg gcccgggccc gggccc          56

<210> 230
<211> 56
<212> DNA
<213> Artificial Sequence

<220>
<223> Sequence with address part and decoding information data part of
      a second example of sequences comprising information represented
      using a graphical representation, as shown in line 35 of Fig. 4c

<400> 230
ggactgcagg gcccgtgccc gggcccgggc ccgggcccgg gcccgggccc gggccc          56


<210> 231
<211> 56
<212> DNA
<213> Artificial Sequence

<220>
<223> Sequence with address part and decoding information data part of
      a second example of sequences comprising information represented
      using a graphical representation, as shown in line 36 of Fig. 4c

<400> 231
ttcctgcagg gcccgggccc gggcccgggc ccgggcccgg gcccgggccc gggccc          56


<210> 232
<211> 56
<212> DNA
<213> Artificial Sequence

<220>
<223> Sequence with address part and decoding information data part of
      a second example of sequences comprising information represented
      using a graphical representation, as shown in line 37 of Fig. 4c

<400> 232
gtcctgcagg gcccgggccc gggcccgggc ccgggcccgg gcccgggccc gggccc          56


<210> 233
<211> 56
<212> DNA
<213> Artificial Sequence

<220>
<223> Sequence with address part and decoding information data part of
      a second example of sequences comprising information represented
      using a graphical representation, as shown in line 38 of Fig. 4c

<400> 233
tgcctgcagg gcccgggccc gggcccgggc ccgggcccgg gcccgggccc gggccc          56


<210> 234
<211> 56
<212> DNA
<213> Artificial Sequence

<220>

<223> Sequence with address part and decoding information data part of
      a second example of sequences comprising information represented
      using a graphical representation, as shown in line 39 of Fig. 4c

<400> 234
ggcctgcagg gcccgggccc gggcccgggc ccgggcccgg gcccgggccc gggccc        56


<210> 235
<211> 56
<212> DNA
<213> Artificial Sequence

<220>
<223> Sequence with address part and decoding information data part of
      a second example of sequences comprising information represented
      using a graphical representation, as shown in line 40 of Fig. 4c

<400> 235
ttaaggcagg gcccgggccc gggcccgggc ccgggcccgg gcccgggccc gggccc        56


<210> 236
<211> 56
<212> DNA
<213> Artificial Sequence

<220>
<223> Sequence with address part and decoding information data part of
      a second example of sequences comprising information represented
      using a graphical representation, as shown in line 41 of Fig. 4c

<400> 236
gtaaggcagg gcccgggccc gggcccgggc ccgggcccgg gcccgggccc gggccc        56

## Description

### FIELD

The present disclosure is related to an alternative storage/transmission application, where a plurality of copies of data packets exists. An example for such an application is the archiving of digital data by synthesizing, i.e. artificially creating nucleic acid sequences, such as DNA molecules. More particularly, the present principles relate to the storing of user data, i.e. archival data, and a corresponding decoding manual, i.e. information for decoding said user data, in synthesized nucleic acid sequences, i.e. oligonucleotides or oligos.

### BACKGROUND

An example for an application where a plurality of (possibly disturbed) copies of (initially identical) data packets exists is the archiving of digital data with synthetic, i.e. artificially generated DNA. Storing data in sequences of nucleotides of artificially generated DNA molecules has been investigated in "Next-generation digital information storage", Church et al., Science 337, 1628, 2012 [I], and in "Towards practical, high-capacity, low-maintenance information storage in synthesized DNA", Goldman et al., Nature, vol. 494, 2013 [II]. DNA as storage medium exhibits desired storage properties, such as longevity, huge storage density, and low-cost for maintenance.

Nucleic acid sequences, such as strands of DNA (Deoxyribonucleic Acid) or RNA (Ribonucleic Acid) fragments (oligonucleotides, short: oligos), can be generated or synthesized, using a nucleic acid synthesizer, as sequences of four different nucleotides identified by their respective nucleobases, namely Adenine, Thymine, Cytosine and Guanine, shortly denoted as A, T, C and G, for DNA, or, respectively, Adenine, Uracil, Cytosine and Guanine, shortly denoted as A, U, C and G, for RNA. The order of the nucleotides forming the oligo is used to store code symbol sequences, for example sequences of quaternary code symbols, representing user data, for example provided as sequences of binary or quaternary code symbols. Nucleic acid sequencers are used to readout the sequence of nucleotides, i.e. to transform an oligo back into its corresponding code symbol sequence. The described biochemical processing usually requires multiple copies of the same oligo in order to function. Therefore, the processing comprises an amplification of the oligos, i.e. multiple duplication of the oligos, for example using Polymerase Chain Reaction (PCR).

DNA strands have proven to be very stable even over long time periods, so information stored in DNA is expected to be readable after hundreds, thousands or even millions of years. Nevertheless, the source or user data have to be encoded and/or modulated in a specific way. This comprises, e.g., how the user data are distributed to different packets, what kind of error protection is used etc. Therefore, for decoding the user data, it is necessary to know how the encoding was done. Hence, decoding information, i.e. a "manual", is needed that describes, how the decoding can be performed and, e.g., how the encoding was done.

Said decoding information or manual could be provided on a separate medium (e.g. on paper, a harddisc etc.), and stored near the nucleic acid storage container having stored therein the DNA strands carrying the user data encoded in their sequence of nucleotides. However, when targeting long preservation durations, there is no guarantee that the manual will survive as long as the DNA strands or that the technology for reading the storage medium for the manual will be available anymore, or that the DNA storage and the manual will still be available in combination, i.e. that the decoding manual will not have been disjoint from the DNA storage in the meantime.

On the other hand, DNA as the substance that is carrying the biochemical information of living organisms will still be available, and there is a high probability that technologies for processing DNA will remain available, for example for sequencing, i.e. retrieving the sequences of nucleotides within DNA.

There remains a need to preserve the decoding information, i.e. the manual, how to decode user data stored in synthesized DNA, over the same long time as the user data itself, identifiable as being the decoding information, and without losing the connection between the stored user data and its decoding information.

### SUMMARY

A method for storing user data (e.g. archival data) and decoding information data in subsets of a plurality of synthesized oligos is provided, wherein the user data are stored together with the related decoding information data in a plurality of oligos. Further, a corresponding apparatus and a substance comprising at least said subsets are provided.

According to one aspect of the present principles, a method for storing user data and decoding information data in subsets of a plurality of synthesized oligos comprises
- obtaining user data and decoding information data required for decoding the user data;
- creating a first subset of code symbol sequences having encoded said user data and a second subset of code symbol sequences having encoded said decoding information data;
- synthesizing a corresponding first subset of oligos from said first subset of code symbol sequences and a corresponding second subset of oligos from said second subset of code symbol sequences using a nucleic acid synthesizer device, wherein
- the second subset of oligos contains less oligos than the first subset of oligos, and
- the oligos of the second subset of oligos exhibit at least one specific property not occurring in any oligo of the first subset of oligos.

The first subset and the second subset are then stored together, for example in a nucleic acid storage container.

The decoding information can comprise all information required to perform the actual decoding. In one embodiment it may, for example, comprise an information identifying an encoding scheme rather than the complete encoding scheme itself.

The term "specific property" does not solely refer to a different content, but comprises at least one structural difference between the oligos of the second subset as compared to the oligos of the first subset, such as a different primer sequence, oligo length or a certain nucleotide pattern used when constructing the sequence of nucleotides.

"Synthesizing" an oligo refers to generating or creating an oligo, i.e. a nucleotide sequence, by transforming a code symbol sequence into a corresponding nucleotide sequence using a nucleic acid synthesizer device, such as a DNA synthesizer.

A code symbol sequence is a set of subsequent code symbols, e.g., in the form of an electronic signal, for example quaternary code symbols with each code symbol value corresponding to a nucleotide type. As another example, a code symbol sequence can also be a sequence of binary code symbols.

User data and decoding information data are both part of payload data stored in a payload/data part of an oligo. Further, the oligos comprise an address or index part as well as a primer sequence of nucleotides, which can be used when amplifying an oligo.

In one embodiment, the specific property is introduced when generating the sequences of code symbols to the synthesized as, i.e. to be transformed into, the corresponding oligos. In another embodiment, the specific property is introduced during the synthesizing, i.e. when transforming the sequences of code symbols into sequences of nucleotides.

The term "the second subset of oligos contains less oligos than the first subset" refers to a clear, i.e. a clearly detectable, difference of the number of oligos of the first subset compared to the second subset. In one embodiment, the difference is defined by a threshold, such as the number of oligos of the second subset being at least 5%, 10%, 20%, 30%, 40%, 50%, etc., or 90% or more smaller than the number of oligos contained in the first subset of oligos. In one embodiment, the unequal distribution of oligos of the first and of the second subset of the plurality of oligos is achieved by generating different amounts of first and second code symbol sequences before synthesizing, i.e. transforming the code symbol sequences into, corresponding amounts of oligos. In another embodiment, the unequal distribution is achieved by amplifying the generated oligos of the first subset by a different amplification factor than the oligos of the second subset, thereby creating different amounts of copies of said oligos.

According to one aspect of the present principles, an apparatus for storing user data and decoding information data in subsets of a plurality of synthesized oligos comprises
- an encoding device configured to obtain user data and decoding information data required for decoding the user data, and to encode said user data in a first subset of code symbol sequences and said decoding information data in a second subset of code symbol sequences; and
- a nucleic acid synthesizer device configured to synthesize a corresponding first subset of oligos from said first subset of code symbol sequences and a corresponding second subset of oligos from said second subset of code symbol sequences, wherein
- the second subset of oligos contains less oligos than the first subset of oligos, and
- the oligos of the second subset of oligos exhibit at least one specific property not occurring in any oligo of the first subset of oligos.

An apparatus for storing user data and decoding information data in subsets of a plurality of synthesized oligos comprises an encoding device, such as a computer, or processor and a memory, or other device capable of processing and encoding the data. The apparatus comprises or is connected or connectable to the nucleic acid synthesizer device or module capable of transforming code symbol sequences into corresponding nucleotide sequences, which is connected or connectable to store the oligos, e.g. in a nucleic acid storage container.

According to another aspect of the present principles, a non-transitory program storage device, readable by a computer, tangibly embodies a program of instructions executable by the computer to perform, using a nucleic acid synthesizer device, a method for storing user data and decoding information data in subsets of a plurality of synthesized oligos according to the present principles.

According to another aspect of the present principles, a substance comprises a plurality of synthesized oligos having stored therein user data and decoding information data, wherein
- a first subset of the plurality of synthesized oligos carries user data; and
- a second subset of the plurality of synthesized oligos carries information data required for decoding the user data; and wherein
- the second subset contains less oligos than the first subset, and
- the oligos of the second subset exhibit at least one specific property not occurring in any oligo of the first subset.
The substance comprises the synthesized oligos, for example provided as solid matter or dissolved in a liquid.

The provided solution allows storing of decoding manual information as oligos together with the oligos carrying the user data, but in such a way that it can easily, without prior knowledge of the encoding strategy, be detected that there are two different subsets of oligos contained in the same nucleic acid storage. Once it has been discovered that there is a clearly identifiable second subset contained, the contained information can be accessed.

The provided approach at least has the effect that there is a high probability that the stored manual or decoding information will be identified within the stored oligos. As that privileged information in the second subset can be found, the contained decoding information can be used for decoding of the user data contained in the first subset, even if after a very long time, probably centuries, no separate decoding information is available anymore at the time of decoding. When people in the future will try to decode data stored as sequences of nucleotides, such as DNA strands, they will assume, that the decoding method will also be described somewhere in the archived data. They will first try to find a manual within the plurality of oligos and the subset of the plurality of oligos containing the manual will be identified, as there will be much fewer of them than the total number of DNA strands and they will be provided having specific properties differentiating them from the other oligos.

The specific property to distinguish between the first and second subset of the oligos is chosen such that a person using a sequencer device for retrieving the code symbol sequences from the stored plurality of nucleotide sequences or oligos will easily detect the specific property being different between the oligo subsets or, respectively, their corresponding read out subsets of code symbol sequences.

In one embodiment the at least one specific property comprises an oligo length of the oligos of the second subset of oligos different from any oligo length of the oligos of the first subset of oligos.
The oligo length refers to the number or amount of nucleotides forming the nucleotide sequence or oligo. This property is easy to detect, once the stored oligos are analyzed.

In one embodiment the synthesizing comprises synthesizing each of the oligos of the plurality of oligos having at least one primer sequence, and wherein the at least one specific property comprises a primer sequence in the oligos of the second subset of oligos different from any primer sequence in the oligos of the first subset of oligos. As the primer sequence will have to be read, this differing specific property will also easily be identified. Choosing a different primer as the specific property gives the possibility to amplify the related oligos of the second subset separately from the remaining oligos of the first subset.

In one embodiment the at least one specific property comprises at least one nucleotide pattern in the oligos of the second subset of oligos different from any nucleotide pattern in the oligos of the first subset of oligos. A nucleotide pattern comprises that the oligos of the second subset comprise nucleotides arranged according to a specific pattern that should be discovered or identified with a high probability. For example, the sequences of nucleotides may contain their nucleotides as consecutive pairs or triples of the same nucleotide type.

The manual will be distributed across several oligos. Therefore, in an embodiment an index or address will be used to allow to put the code symbol sequences carrying the decoding information into their correct order. This address part could be identified by an easily detectable encoding scheme different from that used for the data part, i.e. the coding scheme for the addressing part is chosen obviously different from the coding scheme for the manual data.

In one embodiment the creating of the second subset of code symbol sequences comprises creating an address part encoded using a first coding scheme different from a second coding scheme used for encoding a data part of the code symbol sequences. The data part of the code symbol sequences corresponding to the second subset carries, i.e. has encoded, the decoding information.

In one embodiment, the code symbol sequences are generated from quaternary code symbols with two quaternary code symbol values representing a first binary value and two remaining code symbol values representing a second binary value different from the first binary value.This allows a direct mapping of quaternary code symbol values (and thereby nucleotides) and binary code symbol values. For example, quaternary code symbol values can be named "A", "T", "C", and "G" corresponding to the nucleotides of a DNA sequence, and wherein two quaternary code symbol values, e.g. A and T, represent binary code symbol value "0", whereas the remaining code symbol values C and G represent binary code symbol value "1 ".

In one example embodiment, according to the first coding scheme the address part is created by alternatingly concatenating pairs of code symbols of a first group and of a second group, while beginning the address part with a code symbol of the first group, the first group consisting of code symbols of two quaternary code symbol values representing the first binary value and the second binary value, the second group consisting of code symbols of two remaining quaternary code symbol values different from the two code symbol values of the first group and also representing the first binary value and the second binary value, and
- according to the second coding scheme the data part is created by alternatingly concatenating pairs of code symbols of the second group and of the first group, while beginning the data part with a code symbol of the second group. The change of the coding scheme will be easily detectable when analyzing retrieved code symbol sequences after sequencing the stored oligos, as it will be identifiable as the only position in the sequence where two symbols belonging to the same group will follow each other directly, i.e. where the scheme of alternating symbol groups does not apply. For example, the first group may consist of code symbols with values "A" and "C", whereas the second group consists of code symbols with values "T" and "G". Example code symbol sequences "ATCGATCGTAGCTCGAGA" and "CGATCGATGCGATCTAGC" then exhibit the following coding scheme exception: At the ninth position a quaternary code symbol of the group T/G follows a quaternary code symbol of the same group - only there.

In another example embodiment, according to the first coding scheme the address part is created by alternatingly concatenating a first number of code symbols of a first group with a same first number of code symbols of a second group, the first group consisting of code symbols of two quaternary code symbol values representing the first binary value and the second binary value, the second group consisting of code symbols of two remaining quaternary code symbol values different from the two code symbol values of the first group and also representing the first binary value and the second binary value, and
- according to the second coding scheme the data part is created by alternatingly concatenating a second number of code symbols of the first group with a same second number of code symbols of the second group, wherein the second number differs from the first number. The change of the coding scheme will be easily detectable when analyzing retrieved code symbol sequences after sequencing the stored oligos, as it will be identifiable as the only position in the sequence where the scheme of a first amount of symbols, e.g. two, belonging to the same group followed by the same amount of symbols belonging the other group changes to a different scheme where a second amount of symbols, e.g. three, belonging to the same group is followed by the same amount of symbols belonging the other group. For example, the first group may consist of code symbols with values "A" and "C", whereas the second group consists of code symbols with values "T" and "G". If, for example, the used first number equals two and the second number equals three, example code symbol sequences, such as "TGAAGTCATGTACCTTTACA" and "GGACTTACTTGAACGTGAAA", exhibit a detectable position, where the coding scheme changes.

The manual data or decoding information data part contains the manual information encoded in a straightforward way. In one embodiment the oligos of the second subset of oligos contain the information for decoding the user data as a graphical representation. According to the embodiment, the graphical representation becomes visible after sequencing the oligos of the second subset and, for example, displaying the retrieved corresponding code symbol sequences. In an embodiment, the graphically represented information becomes visible to a user (e.g. on a display screen or printout) after sorting the obtained code symbol sequences according to their (increasing) addresses encoded in the address parts.

According to an example embodiment, like in black and white images, a "1" represents a white pixel, while a "0" represents a black one (or vice versa). A corresponding approach works with quaternary code symbols corresponding to the four nucleotides. Although the graphical data are encoded in a specific way, the correctly sorted code symbol sequences (according to the address or index) for the corresponding oligos already show graphical patterns, which are an indication that the data part contains graphical information.

There is a high probability that the manual or decoding information stored according to the present principles will be identified within the stored oligos and will be decodable even without prior knowledge about the coding scheme. This will allow decoding of the data even after very long preservation durations.

While not explicitly described, the presented embodiments may be employed in any combination or sub-combination.

### BRIEF DESCRIPTION OF THE DRAWINGS

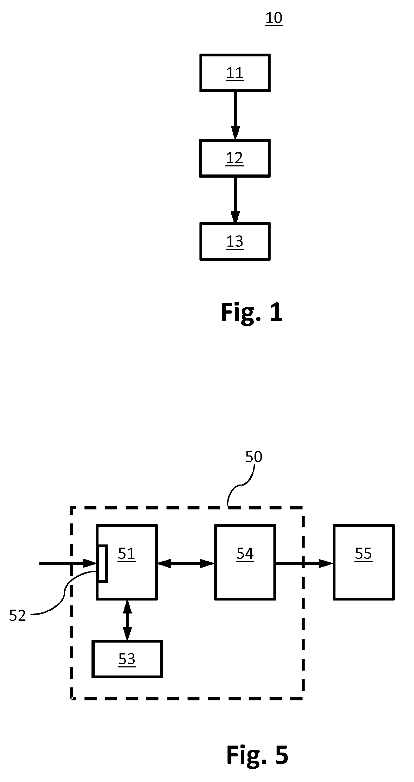

Fig. 1 schematically illustrates an example of an embodiment of a method for storing user data and decoding information data in subsets of a plurality of synthesized oligos;

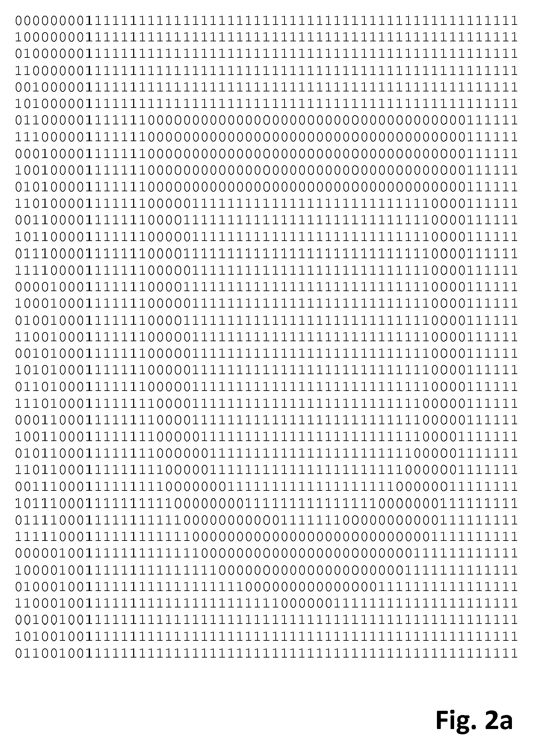

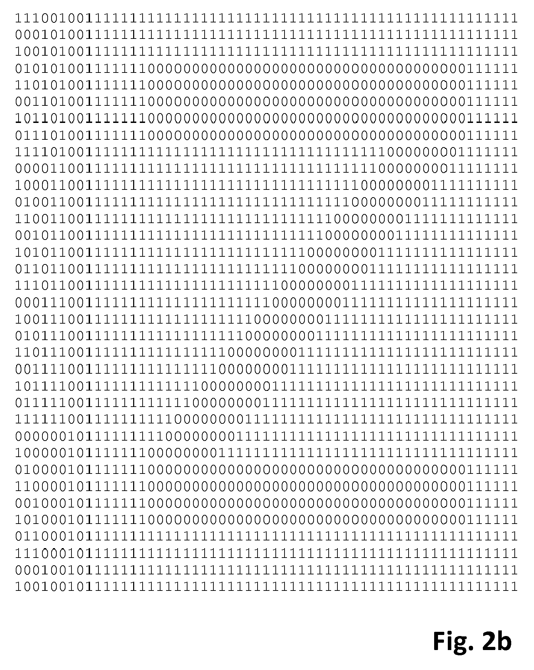

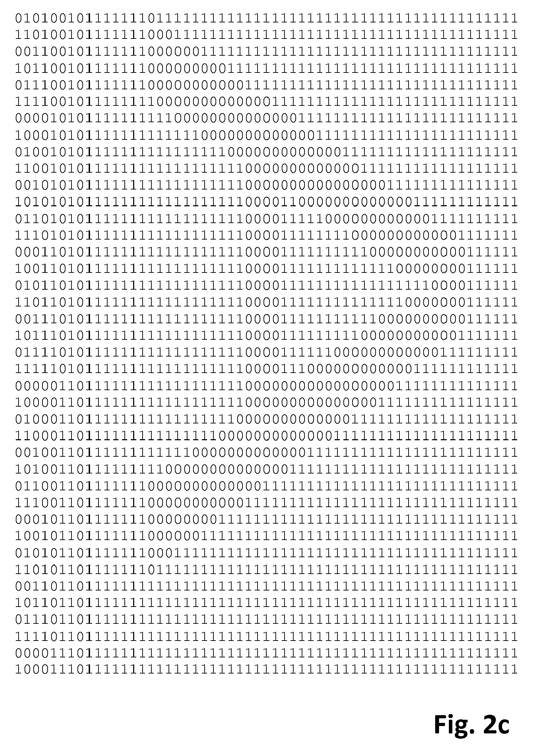

Fig. 2a, 2b and 2c schematically illustrate a first, a second and third part of an example of a plurality of binary encoded code symbol sequences comprising an address data part and a decoding information data part comprising information represented using a graphical representation, to be used for synthesizing oligos;

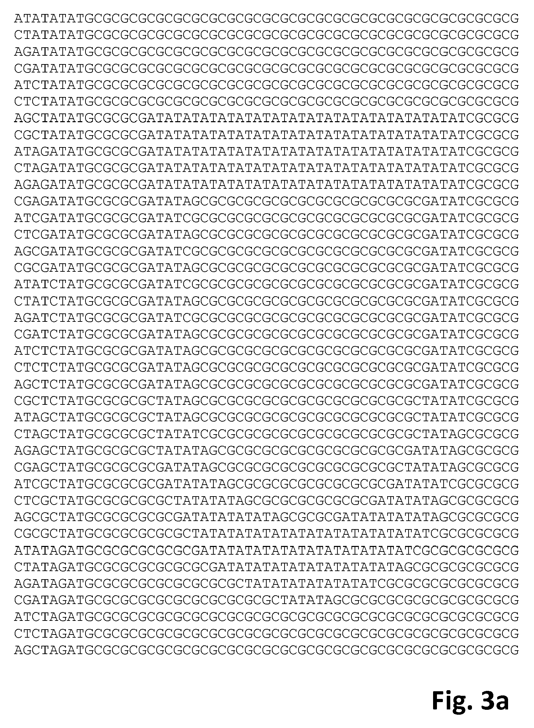

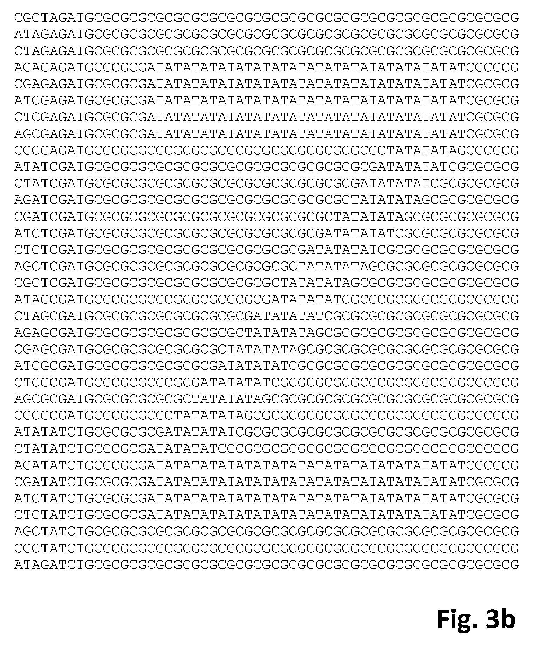

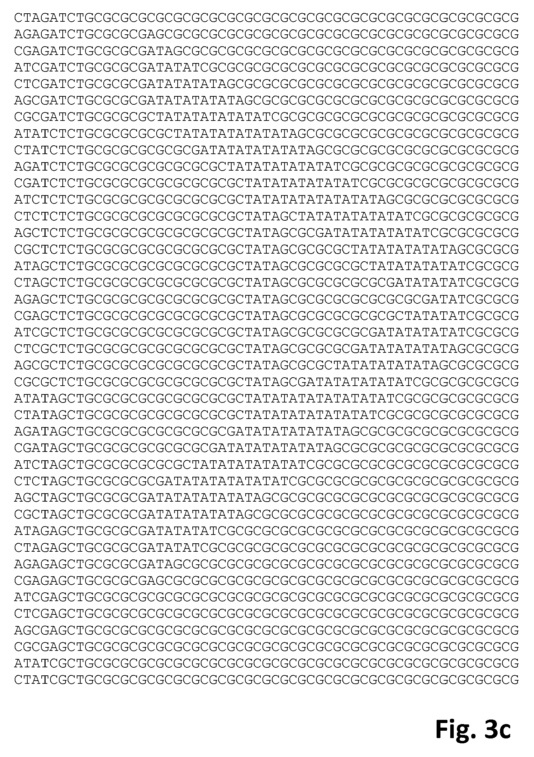

Fig. 3a, 3b and 3c schematically illustrate a first, a second and third part of a first example of a plurality of quaternary encoded code symbol sequences comprising an address data part and a decoding information data part comprising information represented using a graphical representation, to be used for synthesizing oligos;

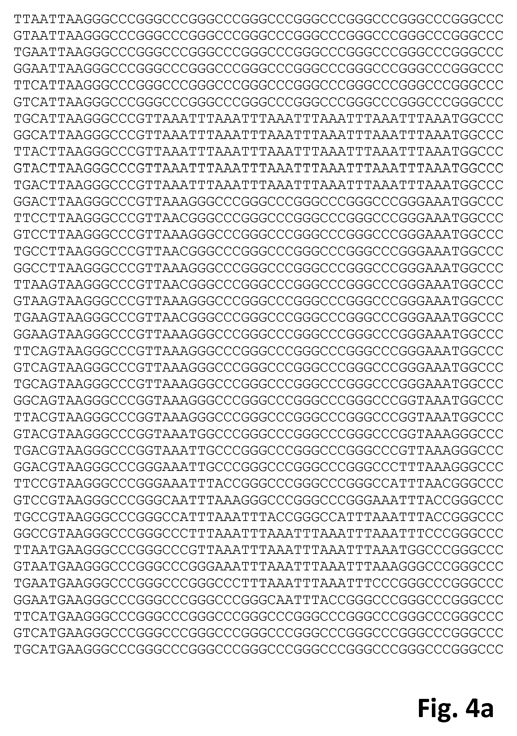

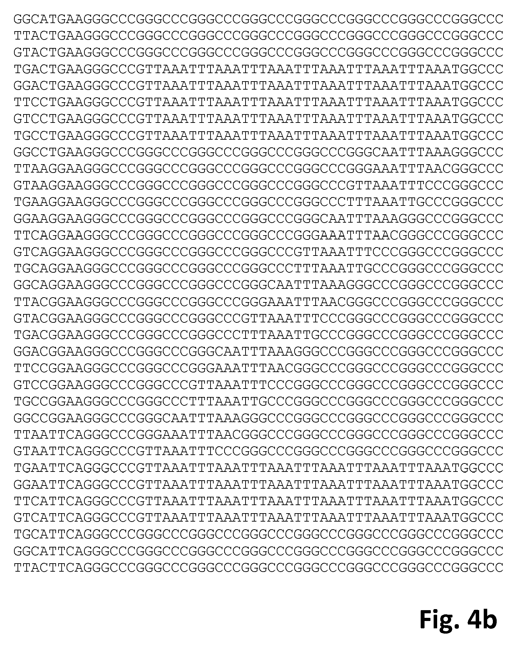

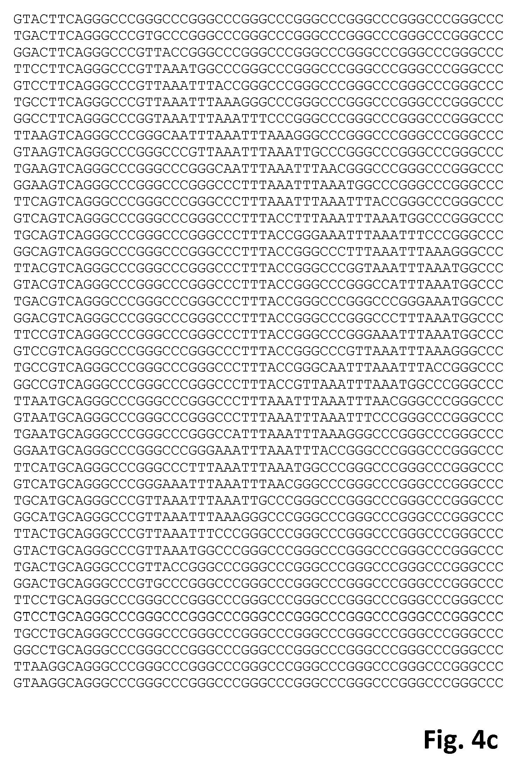

Fig. 4a, 4b and 4c schematically illustrate a first, a second and third part of a second example of a plurality of quaternary encoded code symbol sequences comprising an address data part and a decoding information data part comprising information represented using a graphical representation, to be used for synthesizing oligos; and
Fig. 5 schematically illustrates an example of an embodiment of an apparatus for storing user data and decoding information data in subsets of a plurality of synthesized oligos.

### DETAILED DESCRIPTION OF EMBODIMENTS

For a better understanding of the principles, example embodiments are explained in more detail in the following description with reference to the figures. It is understood that the present solution is not limited to these exemplary embodiments and that specified features can also expediently be combined and/or modified without departing from the scope of the present principles as defined in the appended claims.

Referring to Fig. 1, an example of an embodiment of a method 10 for storing user data and decoding information data in subsets of a plurality of synthesized oligos is schematically illustrated.

In a first step 11 user data and decoding information data required for decoding the user data are obtained, for example from a data transmission channel or data storage.

In a next step 12 a first subset of code symbol sequences having encoded said user data and a second subset of code symbol sequences having encoded said decoding information data are created.

In a next step 13 a corresponding first subset of oligos is synthesized from said first subset of code symbol sequences and a corresponding second subset of oligos is synthesized from said second subset of code symbol sequences using a nucleic acid synthesizer device.

The code symbol sequence creation step 12 and/or the synthesizing step 13 are adapted such that the second subset of oligos contains less oligos than the first subset of oligos, and that the oligos of the second subset of oligos exhibit at least one specific property not occurring in any oligo of the first subset of oligos.

Further, as the manual or decoding information is distributed over the oligos of the second subset, an address or index is included in the decoding information which allows for sorting the retrieved code symbol sequences corresponding to the oligos of the second subset correctly after sequencing.

According to an embodiment, the content of the manual or decoding information is encoded in the data part of the oligo using a graphical representation: Like in black and white images a 1 stands for a white pixel, while a 0 for a black one (or vice versa). Referring to Fig. 2A, Fig. 2B and Fig. 2C, a first, a second and third part of an example of a plurality of binary encoded code symbol sequences comprising an address data part and a decoding information data part comprising information represented using a graphical representation, to be used for synthesizing oligos, are shown. The example illustrates the three letters "D", "N", "A" encoded as a graphical representation and serving as an example for any decoding information that could be stored in the second subset of the plurality of oligos. Each line represents the bits encoded in one code symbol sequence to be used for synthesizing a corresponding oligo. The first eight bits form the address or index, while the remaining 49 bits contain the data.

As described above, a code symbol sequence and, respectively, a corresponding oligo will comprise an address part and a data part. This partitioning into two parts is made detectable by using different coding schemes for the two parts.

Referring to Fig. 3A, Fig. 3B and Fig. 3C, a first, a second and third part of a first example of a plurality of quaternary encoded code symbol sequences comprising an address data part and a decoding information data part comprising information represented using a graphical representation, to be used for synthesizing oligos, is illustrated. Each line represents one quaternary code symbol sequence to be used for synthesizing a corresponding oligo. For the shown example, quaternary code symbol sequences consisting of code symbols A, T, C and G, e.g. corresponding to the four types of nucleotides of a DNA sequence, are generated. The shown example is derived from the example shown in Figs. 2A, 2B and 2C, wherein the binary code symbol "0" is encoded using quaternary code symbol "A" or "T" and the binary code symbol "1" is encoded using quaternary code symbol "C" or "G". Here, the code symbols A/C and T/G corresponding to nucleotides are used in alternating order. As an exception from that scheme, at one position within the code symbol sequence, there is no alteration. For example, the code symbol sequence "ATCGATCGTAGCTCGAGA" and "CGATCGATGCGATCTAGC" exhibit the following coding scheme exception: At the ninth position a quaternary code symbol of the group T/G follows a quaternary code symbol of the same group - only there. This can be discovered as an indication that this is a specific position partitioning the oligo into two parts. Further, choosing quaternary code symbols from different groups in an alternating manner also prohibits a runlength of more than three identical nucleotides when transforming said code symbol sequence into its corresponding nucleotide sequence, which might otherwise lead to problems in the nucleic acid sequencer. Again, the example illustrates that the three letters "D", "N", "A" remain visibly detectable as a graphical representation.

Referring to Fig. 4A, Fig. 4B and Fig. 4C, a first, a second and third part of a second example of a plurality of quaternary encoded code symbol sequences comprising an address data part and a decoding information data part comprising information represented using a graphical representation, to be used for synthesizing oligos, is illustrated. Each line represents one quaternary code symbol sequence to be used for synthesizing a corresponding oligo. For the shown example, again quaternary code symbol sequences consisting of code symbols A, T, C and G, e.g. corresponding to the four types of nucleotides of a DNA sequence, are generated. The shown example is also derived from the example shown in Figs. 2A, 2B and 2C, wherein the binary code symbol "0" is encoded using quaternary code symbol "A" or "T" and the binary code symbol "1" is encoded using quaternary code symbol "C" or "G". In the shown example, in a first part of the code symbol sequence, always two quaternary code symbols of the first group T/G to be transformed into corresponding nucleotides of type T/G are followed by two quaternary code symbols of the second group A/C to be transformed into a corresponding nucleotides of type A/C, while in a second part, three quaternary code symbols of the first group are followed by three quaternary code symbols of the second group. This coding scheme has also been applied in the following example code symbol sequences: "TGAAGTCATGTACCTTTACA" and "GGACTTACTTGAACGTGAAA".

The usage of groups of different length clearly separates the code symbol sequence and, therefore, the oligo synthesized from said code symbol sequence, in two parts containing different information. By limiting the group length to three, a runlength of more than three identical nucleotides is prevented.

In order to provide an intuitive scheme that can be easily identified, the groups have been selected as described, as the corresponding nucleotides A and T resp. C and G are complementary to each other. It should be noted, that it is also possible to use different groups for representing "0" and "1" (e.g. "A" and "C" for "0", "T" and "G" for "1 ").

The index or address/ID, which can be a contiguous number, can be encoded in the address part of the oligo as a standard binary number. In an embodiment it is determined if a MSB (most significant bit) first or LSB (least significant bit) first approach has been chosen: Following a statistical analysis on the different address bits, the MSB and LSB are found: For the LSB the probabilities of "0" and "1" are nearly identical, while for the MSB the probability of a "0" is much higher than the probability of a "1 ".

This property can also be used to determine which code symbols, respectively nucleotides, were chosen to represent which binary value. Further, as the probability for a "0" is growing in a monotonous way from the LSB (where it is 0.5) to the MSB, the probability distribution is an indication that binary numbers were encoded in this part of the oligo.

As shown in Figs. 3A, 3B, 3C and Figs. 4A, 4B, 4C, although the graphical data are encoded in a specific way, the correctly sorted code symbol sequences (according to the address or index) already show graphical patterns "D", "N", "A".

Storing graphics is an intuitive approach that will be understood in the future. In other embodiments, for example specific text encoding algorithms (ASCII, UTF-8 etc.) can be used to store single letters in groups of bits, which results in a much smaller amount of oligos needed to represent the manual. The detection and decoding will be possible, if the chosen text encoding is still known at the time of decoding. In yet another embodiment both approaches are combined: In an initial graphical part the encoding is described that is used later.

In another embodiment targeting at archiving for very long time periods, a manual as stored decoding information starts with a (graphical) description of the chosen language, as the language may probably not be known anymore at the time of decoding.

When using the described storing method, there is a specific probability that single nucleotides are distorted during the process. In an embodiment, a correction mechanism is applied, e.g. performing of majority decisions between oligos of a same address. However, even without a correction, due to the graphical representation the result will most often remain readable for the human reader.

Referring to Fig. 5, an example of an embodiment of an apparatus 50 for storing user data and decoding information data in subsets of a plurality of synthesized oligos is schematically illustrated. The apparatus 50 allows implementing the advantages and characteristics of the described method as part of an apparatus for storing user data and decoding information data in subsets of a plurality of synthesized oligos.

The apparatus 50 comprises an encoding device 51 configured to obtain user data and decoding information data required for decoding the user data, and to encode said user data in a first subset of code symbol sequences and said decoding information data in a second subset of code symbol sequences. In the shown example, the decoding device 51 comprises an interface 52 for obtaining the user data and decoding information data as input from a data transmission channel or storage medium. Further, the decoding device 51 is connected to (or comprises) a memory 53 for storing the obtained input data and encoded code symbol sequences.

The apparatus 50 further comprises a nucleic acid synthesizer device 54, connected to receive the first and second subsets of code symbol sequences from the encoding device 51 and configured to synthesize a corresponding first subset of oligos from said first subset of code symbol sequences and a corresponding second subset of oligos from said second subset of code symbol sequences, wherein
- the second subset of oligos contains less oligos than the first subset of oligos, and
- the oligos of the second subset of oligos exhibit at least one specific property not occurring in any oligo of the first subset of oligos.

The shown nucleic acid synthesizer device 54 is connected to a nucleic acid storage container 55 for storing a substance comprising the plurality of synthesized oligos of the first and the second subsets. In other embodiments, the nucleic acid synthesizer device 54 it is connectable to or comprises the storage container 55.

In the example embodiment shown in Fig. 5 the shown encoding device 51 and the nucleic acid synthesizer device 52 directly communicate with each other. In another embodiment the apparatus 50 comprises a controller device connected to one or more of the shown devices and controls their communication.

Depending on the embodiment, the encoding device 51 and the nucleic acid sequencer device 54 are provided as separate devices or jointly as one device. In one embodiment, the encoding device 51 is provided as (functionality carried out or implemented by) a processor, microprocessor, microcontroller or other processing device, computer or other programmable apparatus or processor assembly connected to or comprising the memory 53, and the memory device 53 stores instructions that, when executed, cause the apparatus to perform a method according to the present principles.

For this, (a part of) the shown memory device 53 can be a non-transitory program storage device, tangibly embodying a program of instructions executable by a processor.

Aspects of the present principles can be embodied as a method, an apparatus, a system, a computer program product or a computer readable medium, i.e. aspects of the present principles may be implemented in various forms of hardware, software, firmware, special purpose processors, or a combination thereof. Accordingly, aspects of the present principles can take the form of a hardware embodiment, a software embodiment or an embodiment combining software and hardware aspects. Aspects of the present principles may, for example, at least partly be implemented in a computer program comprising code portions for performing steps of the method according to an embodiment of the present principles when run on a programmable apparatus or enabling a programmable apparatus to perform functions of an apparatus, device or system according to an embodiment of the present principles. Moreover, the software is preferably implemented as an application program tangibly embodied on a program storage device. The application program may be uploaded to, and executed by, a machine comprising any suitable architecture. Preferably, the machine is implemented on a computer platform having hardware such as one or more processors/central processing units (CPU), a random access memory (RAM), and input/output (I/O) interface(s). The computer platform also includes an operating system and microinstruction code. Various processes and functions described herein may either be part of the microinstruction code or part of the application program (or a combination thereof), which is executed via the operating system. In addition, various other devices may be connected to the computer platform such as the nucleic acid synthesizer device 54, a nucleic acid storage container 55, but also, e.g. an additional data storage device and a printing device.

Unless stated otherwise, terms such as "first" and "second" are used to arbitrarily distinguish between the elements the terms describe and are not necessarily intended to indicate temporal or other prioritization of the elements. Any connection shown may be a direct connection or an indirect connection. Further, those skilled in the art will recognize that the boundaries between logic blocks are merely illustrative and that alternative embodiments may merge logic blocks or impose an alternate decomposition of functionality upon various logic blocks.

### CITATION LIST

[I] George M. Church, Yuan Gao, Sriram Kosuri, "Next-Generation Digital Information Storage in DNA", Science Vol. 337, 28 September 2012.
[II] Nick Goldman et al., "Towards practical, high-capacity, low-maintenance information storage in synthesized DNA", Nature Vol. 494, January 2013.

## Claims

1. A method (10) for storing user data and decoding information data in subsets of a plurality of synthesized oligos, comprising
- obtaining (11) user data and decoding information data required for decoding the use data;
- creating (12) a first subset of code symbol sequences having encoded said user data and a second subset of code symbol sequences having encoded said decoding information data;
- synthesizing (13) a corresponding first subset of oligos from said first subset of code symbol sequences and a corresponding second subset of oligos from said second subset of code symbol sequences using a nucleic acid synthesizer device, wherein
- the second subset of oligos contains less oligos than the first subset of oligos, and
- the oligos of the second subset of oligos exhibit at least one specific property not occurring in any oligo of the first subset of oligos.

2. The method according to claim 1, wherein the at least one specific property comprises an oligo length of the oligos of the second subset of oligos different from any oligo length of the oligos of the first subset of oligos.

3. The method according to claim 1 or claim 2, wherein the synthesizing (13) comprises synthesizing each of the oligos of the plurality of oligos having at least one primer sequence, and wherein the at least one specific property comprises a primer sequence in the oligos of the second subset of oligos different from any primer sequence in the oligos of the first subset of oligos.

4. The method according to any of the preceding claims, wherein the at least one specific property comprises at least one nucleotide pattern in the oligos of the second subset of oligos different from any nucleotide pattern in the oligos of the first subset of oligos.

5. The method according to any of the preceding claims, wherein the creating of the second subset of code symbol sequences comprises creating an address part encoded using a first coding scheme different from a second coding scheme used for encoding a data part of the code symbol sequences.

6. The method according to claim 5, wherein the code symbol sequences are generated from quaternary code symbols with two quaternary code symbol values representing a first binary value and two remaining code symbol values representing a second binary value different from the first binary value.

7. The method according to claim 6, wherein
- according to the first coding scheme the address part is created by alternatingly concatenating pairs of code symbols of a first group and of a second group, while beginning the address part with a code symbol of the first group, the first group consisting of code symbols of two quaternary code symbol values representing the first binary value and the second binary value, the second group consisting of code symbols of two remaining quaternary code symbol values different from the two code symbol values of the first group and also representing the first binary value and the second binary value, and wherein
- according to the second coding scheme the data part is created by alternatingly concatenating pairs of code symbols of the second group and of the first group, while beginning the data part with a code symbol of the second group.

8. The method according to claim 6, wherein
- according to the first coding scheme the address part is created by alternatingly concatenating a first number of code symbols of a first group with a same first number of code symbols of a second group, the first group consisting of code symbols of two quaternary code symbol values representing the first binary value and the second binary value, the second group consisting of code symbols of two remaining quaternary code symbol values different from the two code symbol values of the first group and also representing the first binary value and the second binary value, and wherein
- according to the second coding scheme the data part is created by alternatingly concatenating a second number of code symbols of the first group with a same second number of code symbols of the second group, wherein the second number differs from the first number.

9. The method according to any of the preceding claims, wherein the oligos of the second subset of oligos contain the information for decoding the user data as a graphical representation.

10. An apparatus (50) for storing user data and decoding information data in subsets of a plurality of synthesized oligos, comprising
- an encoding device (51) configured to obtain user data and decoding information data required for decoding the user data, and to encode said user data in a first subset of code symbol sequences and said decoding information data in a second subset of code symbol sequences; and
- a nucleic acid synthesizer device (54) configured to synthesize a corresponding firstsubset of oligos from said first subset of code symbol sequences and a corresponding second subset of oligos from said second subset of code symbol sequences, wherein
- the second subset of oligos contains less oligos than the first subset of oligos, and
- the oligos of the second subset of oligos exhibit at least one specific property not occurring in any oligo of the first subset of oligos.

11. A substance comprising
a plurality of synthesized oligos having stored therein user data and decoding information data, wherein
- a first subset of the plurality of synthesized oligos carries user data; and
- a second subset of the plurality of synthesized oligos carries information data required for decoding the user data; and wherein
- the second subset contains less oligos than the first subset, and
- the oligos of the second subset exhibit at least one specific property not occurring in any oligo of the first subset.

12. The substance according to claim 11, wherein the at least one specific property comprises an oligo length of the oligos of the second subset of oligos different from any oligo length of the oligos of the first subset of oligos.

13. The substance according to claim 11 or claim 12, wherein each of the oligos of the plurality of oligos comprises at least one primer sequence and the at least one specific property comprises a primer sequence in the oligos of the second subset of oligos different from any primer sequence in the oligos of the first subset of oligos.

14. The substance according to any of claims 11 to 13, wherein the at least one specific property comprises at least one nucleotide pattern in the oligos of the second subset of oligos different from any nucleotide pattern in the oligos of the first subset of oligos.

15. The substance according to any of claims 11 to 14, wherein the oligos of the second subset of oligos contain the information for decoding the user data as a graphical representation.
